(19) Europäisches Patentamt / European Patent Office / Office européen des brevets

(11) **EP 2 492 681 A1**

(12) **EUROPEAN PATENT APPLICATION**

(43) Date of publication:
29.08.2012 Bulletin 2012/35

(21) Application number: **12155155.0**

(22) Date of filing: **09.09.2004**

(51) Int Cl.:
*G01N 31/00* (2006.01)    *G01N 33/00* (2006.01)
*G01N 35/00* (2006.01)    *B01L 3/00* (2006.01)

(84) Designated Contracting States:
**AT BE BG CH CY CZ DE DK EE ES FI FR GB GR HU IE IT LI LU MC NL PL PT RO SE SI SK TR**

(30) Priority: **09.09.2003 US 501746 P**

(62) Document number(s) of the earlier application(s) in accordance with Art. 76 EPC:
**10165755.9 / 2 275 810**
**04783590.5 / 1 671 117**

(71) Applicant: **BioGenex Laboratories
Fremont, CA 94538 (US)**

(72) Inventor: **Kalra, Krishan
Danville, CA 94506 (US)**

(74) Representative: **Sands, Howard Simon et al
Boult Wade Tennant
Verulam Gardens
70 Gray's Inn Road
GB-London WC1X 8BT (GB)**

Remarks:
•This application was filed on 13-02-2012 as a divisional application to the application mentioned under INID code 62.
•Claims filed after the date of filing of the application/ after the date of receipt of the divisional application (Rule 68(4) EPC).

(54) **Sample processing system**

(57) An apparatus comprising a sample processing system (600) is disclosed. The sample processing system (600) is adapted to perform independent, environmentally-controlled processing for samples on multiple substrates using different processing protocols simultaneously.

FIG.7

**EP 2 492 681 A1**

**Description**

## BACKGROUND OF THE INVENTION

Over the past decade, researchers have developed molecular technologies for disease diagnoses that analyze proteins and DNA/RNA messages that encode them. These developments have facilitated new insights into the causes of disease and into the early detection of diseases and the accompanying potential therapeutic response. Through genomics, scientists have determined that chromosomal and genetic abnormalities are fundamental sources of human disease. Chromosomal and genetic abnormalities encompass a broad range of irregularities, including numerical and structural changes in chromosomes, amplifications and deletions of genes, as well as mutations within specific gene sequences. Scientific evidence suggests that these chromosomal changes are integral to cancer progression and are the most significant markers of cancer detection. Molecular diagnostic laboratories have long used archaic, manual, and cumbersome techniques that often lead to poorly reproducible and inaccurate results. Even today, most molecular and cell-based diagnostic systems use outdated and non-integrated technologies unable to cost-effectively perform massively parallel-scale analyses. System capabilities are further stressed by the genomics revolution that has accelerated demand for potential markers for use in target validation in drug discovery and development. Consequently, additional automation and parallelism are sought to enable efficient specimen handling, processing and analysis.

## SUMMARY

In accordance with an embodiment of a system for handling and processing chemical and/or biological samples, a MieroChamber comprises a substrate, a reservoir formed on the substrate for receiving a chemical and/or biological sample, and an encoder such as a barcode or other suitable device. The encoder encodes information describing at least one characteristic of the substrate and/or reservoir.

Various aspects of the invention are defined in the following clauses:

1. An apparatus comprising:

   a substrate;
   a reservoir formed on the substrate for receiving a chemical and/or biological sample; and
   a barcode formed on the substrate and encoding information describing at least one characteristic of the substrate and/or reservoir.

2. The apparatus according to clause 1 wherein:

   the barcode further encodes information describing at least one characteristic of a sealing device and/or sealing agent.

3. The apparatus according to clause 1 wherein:

   the barcode encodes at least one information item selected from among a group of information items comprising reservoir volume, reservoir size, reservoir shape, reservoir depth, number of reservoirs, substrate material, substrate electrical characteristics, substrate color, and presence and/or properties of components attached to the substrate.

4. The apparatus according to clause 1 further comprising:

   a barrier around the reservoir is a depression etched into the substrate with a shape, size, and depth selected to contain a specified volume.

5. The apparatus according to clause 1 further comprising:

   a barrier formed on a substrate creating the reservoir, the barrier being a shape, size, and height selected to contain a specified volume.

6. The apparatus according to clause 5 wherein:

   the barrier is pre-formed on the substrate before application of the chemical and/or biological sample.

7. The apparatus according to clause 6 wherein:

the barrier is pre-formed of epoxy.

8. The apparatus according to clause 6 wherein:

the barrier is pre-formed of Teflon™ ink.

9. The apparatus according to clause 6 wherein:

the barrier is pre-formed of a hydrophobic material.

10. The apparatus according to clause 5 wherein:

the barrier is formed on a substrate creating a reservoir during processing of a chemical and/or biological sample by a sample processing system.

11. The apparatus according to clause 5 wherein:

the barrier is formed on a substrate holding a pre-applied chemical and/or biological sample by a sample processing system.

12. The apparatus according to clause 1 further comprising:

a plurality of reservoirs formed on the substrate capable of enclosing a plurality of chemical and/or biological sample sections.

13. The apparatus according to clause 11 wherein:

the plurality of reservoirs are the same shape, size, and depth and contain the same volume.

14. The apparatus according to clause 11 wherein:

the plurality of reservoirs vary in shape, size, depth, and/or volume.

15. The apparatus according toclause 1 wherein:

the substrate is a glass slide.

16. The apparatus according to clause 1 wherein:

the substrate is a piezo-electric material.

17. The apparatus according to clause 1 wherein:

the substrate is a thermally-conductive material.

18. The apparatus according to clause 1 wherein:

the substrate is a silicon material.

19. The apparatus according to clause 1 wherein:

the substrate is a polymer material.

20. The apparatus according to clause 1 further comprising:

a cover sealable to the substrate to seal the reservoir.

21. The apparatus according to clause 20 wherein:

the cover is a cap with a size and shape to cover the reservoir.

22. The apparatus according to clause 20 wherein:

the cover is a cover slip with a size and shape to cover the reservoir.

23. The apparatus according to clause 20 wherein:

the cover is constructed from glass.

24. The apparatus according to clause 20 wherein:

the cover is constructed from a plastic or polymer.

25. The apparatus according to clause 20 wherein:

the cover is constructed of a plurality of laminated layers.

26. The apparatus according to clause 20 wherein:

the cover is coated with a conducting material.

27. The apparatus according to clause 20 wherein:

the cover is coated with a conducting material selected from a group comprising metallic paint, liquid metal, metal sheet, metal lamination, and iron foil.

28. The apparatus according to clause 1 further comprising:

a double barrier; and
sealant placed in a location either between barriers of the double barrier, interior to the double barriers, or exterior to the double barrier.

29. An apparatus comprising:

a substrate; and
a barrier formed on the substrate during processing of a chemical and/or biological sample by a sample processing system.

30. The apparatus according to clause 29 further comprising:

a reservoir enclosed by the barrier.

31. The apparatus according to clause 29 wherein:

the barrier is a shape, size, and height selected to contain a specified volume.

32. The apparatus according to clause 29 wherein:

the barrier is a shape, size, and height selected to contain a specified sample.

33. The apparatus according to clause 29 wherein:

the barrier is formed on a substrate holding a pre-applied chemical and/or biological sample.

34. The apparatus according to clause 29 wherein:

the barrier position, size, shape, thickness and/or volume are controllable by the sample processing system.

35. The apparatus according to clause 29 wherein:

the barrier is formed on a substrate holding a pre-applied chemical and/or biological sample and the barrier position, size, shape, thickness and/or volume are controllable by the sample processing system in accordance with directions received via a user interface to form one or more reservoirs corresponding to one or more regions of interest in the chemical and/or biological sample.

36. The apparatus according to clause 29 further comprising:

a plurality of barriers formed on the substrate during processing of a chemical and/or biological sample by a sample processing system.

37. The apparatus according to clause 36 further comprising:

a plurality of reservoirs enclosed by the barriers and capable of enclosing a plurality of chemical and/or biological sample sections.

38. The apparatus according to clause 37 wherein:

the plurality of reservoirs are the same shape, size, and depth and contain the same volume.

39. The apparatus according to clause 37 wherein:

the plurality of reservoirs vary in shape, size, depth, and/or volume.

40. A Micro-chamber comprising:

a substrate;
a reservoir formed on the substrate for receiving a chemical and/or biological sample;
a cover sealable to the substrate to seal the reservoir; and
a vesicle coupled to the cover and containing a reagent.

41. The Micro-chamber according to clause 40 wherein:

the vesicle is dissolvable in contact with a reagent in the reservoir.

42. The Micro-chamber according to clause 40 wherein:

the vesicle is dissolvable by temperature change of the substrate and/or cover.

43. The Micro-chamber according to clause 40 wherein:

the vesicle is predisposed to rupture on contact with the substrate.

44. The Micro-chamber according to clause 40 wherein:

the cover is a cap with a size and shape to cover the reservoir.

45. The Micro-chamber according to clause 40 wherein:

the cover is constructed from glass.

46. The Micro-chamber according to clause 40 wherein:

the cover is constructed from plastic.

47. The Micro-chamber according to clause 40 wherein:

the cover is constructed of a plurality of laminated layers.

48. The Micro-chamber according to clause 40 wherein:

the cover is coated with a conducting material.

49. The Micro-chamber according to clause 40 wherein:

the cover is coated with a conducting material selected from a group comprising metallic paint, liquid metal, metal sheet, metal lamination, and iron foil.

50. The Micro-chamber according to clause 40 wherein:

the cover is a microscope slide.

51. A Micro-chamber comprising:

a substrate;
a reservoir formed on the substrate for receiving a chemical and/or biological sample;
a cover sealable to the substrate to seal the reservoir; and
a vesicle coupled to the substrate and containing a reagent.

52. The Micro-chamber according to clause 51 wherein:

the vesicle is dissolvable in contact with a reagent in the reservoir.

53. The Micro-chamber according to clause 51 wherein:

the vesicle is dissolvable by temperature change of the substrate and/or cover.

54. The Micro-chamber according to clause 51 wherein:

the vesicle is predisposed to rupture on contact with the substrate.

55. An apparatus comprising:

a substrate;
a reservoir formed on the substrate for receiving a chemical and/or biological sample; and
a radio frequency identification (RFID) coupled to the substrate and encoding information describing at least one characteristic of the substrate and/or reservoir.

56. The apparatus according to clause 55 wherein:

the radio frequency identification (RFID) encodes at least one information item selected from among a group of information items comprising reservoir volume, reservoir size, reservoir shape, reservoir depth, number of reservoirs, substrate material, substrate electrical characteristics, substrate color, and presence and/or properties of components attached to the substrate.

57. An apparatus comprising:

a cover adapted to enclose and secure a Micro-chamber containing a chemical and/or biological sample in a reservoir on a substrate; and
a vesicle coupled to the cover and containing a reagent.

58. The apparatus according to clause 57 wherein:

the vesicle is dissolvable in contact with a reagent in the reservoir.

59. The apparatus according to clause 57 wherein:

the vesicle is predisposed to controlled rupture on contact with the substrate.

60. The apparatus according to clause 57 wherein:

the cover is a cap with a size and shape to cover the reservoir.

61. The apparatus according to clause 57 wherein:

the cover is constructed from glass.

62. The apparatus according to clause 57 wherein:

the cover is constructed from plastic.

63. The apparatus according to clause 57 wherein:

the cover is coated with a conducting material.

64. The apparatus according to clause 57 wherein:

the cover is coated with a conducting material selected from a group comprising metallic paint, liquid metal, metal sheet, metal lamination, and iron foil.

65. An apparatus comprising:

a sample processing system adapted to form a Micro-chamber on a substrate, dispense at least one selected reactant to cause reactions in the Micro-chamber, and remove a cover from the Micro-chamber in an automated process.

66. The apparatus according to clause 65 further comprising:

one or more robotic devices that moves relative to the substrate to deliver the at least one selected reactant, and delivers and removes the cover from the Micro-chamber.

67. The apparatus according to clause 65 further comprising:

a platform;
a reagent rack coupled to the platform and adapted to hold a plurality of reagent containers, the containers containing one or more reagents at one or more temperatures.

68. The apparatus according to clause 65 further comprising:

a cover dispenser adapted to dispense covers with a plurality of cover sizes.

69. The apparatus according to clause 65 further comprising:

a substrate processing system adapted to independently maintain a plurality of substrates at different environmental conditions.

70. The apparatus according to clause 65 further comprising:

a substrate processing system adapted to independently maintain a plurality of substrates at different temperatures.

71. The apparatus according to clause 65 further comprising:

a cover dispenser adapted to place a cover on the Micro-chamber and remove the cover from the Micro-chamber.

72. The apparatus according to clause 65 further comprising:

a cover dispenser adapted to place a cover on the Micro-chamber and remove the cover from the Micro-chamber, the cover dispenser being capable of processing covers of multiple different sizes.

73. The apparatus according to clause 65 further comprising:

at least one robotic Z-head adapted to move relative to the substrate.

74. The apparatus according to clause 65 further comprising:

a plurality of attachments including multiple different attachment types adapted for attachment to the at least one robotic Z-head.

75. The apparatus according to clause 65 further comprising:

a plurality of attachments including multiple different attachment types for performing multiple different functions adapted for attachment to the at least one robotic Z-head, the functions being selected from a group comprising gripping and releasing covers, loading and dispensing fluids, loading and dispensing sealant, mixing Micro-chamber contents, washing a Micro-chamber, drying a Micro-chamber.

76. The apparatus according to clause 65 further comprising:

a waste separation system coupled to the sample processing system and configured to divert effluent waste from the sample processing system into a plurality of separate parts.

77. An apparatus comprising:

a sample processing system adapted to concurrently and individually control a plurality of micro-environments within a corresponding plurality of Microchambers on a substrate.

78. The apparatus according to clause 77 further comprising:

a reagent dispensing device configured to apply one or more reagents to the sample;
a cover handling device operable in combination with the reagent dispensing device to automate placement and removal of Micro-chamber covers on the substrate; and
a controller coupled to the sample processing system, the reagent dispensing device, and the cover handling device, the controller being adapted to programmably control the micro-environment by selectively executing a sequence of actions selected from among actions of placing a cover on a Micro-chamber, removing a cover from the Micro-chamber, dispensing a selected reagent to the Micro-chamber; and washing the Micro-chamber.

79. The apparatus according to clause 77 further comprising:

a temperature control assembly with active heating and cooling; and
a controller coupled to the temperature control assembly and adapted to programmably control the micro-environment by selectively applying active heating and cooling.

80. The apparatus according to clause 77 further comprising:

a mixer configured to mix a micro-environment within a Micro-chamber containing a sample on a substrate.

81. The apparatus according to clause 80 further comprising:

a controller coupled to the mixer and adapted to mix Micro-chamber contents under program control.

82. The apparatus according to clause 77 further comprising:

a humidity controller adapted to programmably control humidity in the sample processing system to prevent evaporation in the micro-environment.

83. A method of processing a sample comprising:

forming a Micro-chamber on a substrate;
dispensing at least one selected reactant to cause reactions in the Micro-chamber;
washing the Micro-chamber; and
removing a cover from the Micro-chamber in an automated process.

84. The method according to clause 83 further comprising:

moving one or more robotic devices relative to the substrate;
delivering the at least one selected reactant;
delivering a washing fluid;
delivering the cover to the Micro-chamber; and
removing the cover from the Micro-chamber.

85. The method according to clause 83 further comprising:

providing a plurality of reagents at one or more different temperatures; and
dispensing a selected reagent at a selected temperature to a substrate,

86. The method according to clause 83 further comprising:

dispensing covers with a plurality of cover sizes.

87. The method according to clause 83 further comprising:

independently maintaining a plurality of substrates at different environmental conditions.

88. The method according to clause 83 further comprising:

independently maintaining a plurality of substrates at different temperatures.

89. An apparatus comprising:

a sample processing system configured to apply one or more reagents to a chemical and/or biological sample on a substrate, the sample processing system further comprising:

a robotic device configured to place a cover in a specified position on the substrate and remove the cover from the substrate.

90. The apparatus according to clause 89 further comprising:

a cover dispenser that individually dispenses at least one cover, the dispenser being capable of dispensing covers of multiple different sizes;
a robotic head adapted to move relative to the cover dispenser and the substrate;
an effector coupled to the robotic head that programmably grips and releases the covers, the effector being programmable to perform multiple functions including removing a cover from the cover dispenser, moving the cover to a specified position, placing the cover on the substrate, and removing the cover from the substrate.

91. The apparatus according to clause 90 wherein:

the effector further comprises at least one vacuum pad that grips and releases the covers.

92. The apparatus according to clause 90 wherein:

the effector further comprises an electromagnetic attachment device that grips and releases the covers.

93. The apparatus according to clause 90 wherein:

the effector further comprises a mechanical robotic attachment device that grips and releases the covers.

94. The apparatus according to clause 90 further comprising:

a controller that controls the robotic head and effector; and
a program code executable on the controller and configured to control automatic removal of the covers from the substrate .

95. The apparatus according to clause 90 wherein:

the cover dispenser is stationary and the robotic head is moveable during operation.

96. The apparatus according to clause 90 wherein:

the cover dispenser is coupled to the robotic head and is moveable with the robotic head during operation.

97. The apparatus according to clause90 wherein:

the cover dispenser and the robotic head are moveable relative to one another during operation.

98. The apparatus according to clause90 further comprising:

one or more covers having an epoxy pattern printed on one side to prevent adhering of adjacent covers.

99. The apparatus according to clause90 further comprising:

one or more covers having a chemical repellent coating or electrostatic coating on one or both sides to prevent adhering of adjacent covers.

100. The apparatus according to clause 90 further comprising:

a plurality of robotic heads adapted to move relative to one another and to the substrate.

101. An apparatus comprising:

a sample processing system configured to apply one or more reagents to a chemical and/or biological sample on a substrate, the sample processing system further comprising:

a robotic device configured to create a barrier in a specified position on the substrate.

102. The apparatus according to clause 101 further comprising:

a controller coupled to the robotic device and the sample processing system that programmably creates the barrier on a substrate holding a pre-applied chemical and/or biological sample.

103. The apparatus according to clause 102 wherein:

the controller creates the barrier according to a programmed barrier size, shape, thickness and/or volume.

104. The apparatus according to clause 102 wherein:

the controller creates a plurality of barriers on a single substrate with individually programmable sizes, shapes, thicknesses and/or volumes.

105. The apparatus according to clause 102 wherein:

the controller programmably controls speed of barrier deposition.

106. The apparatus according to clause 102 wherein:

the controller programmably controls positioning of barrier deposition.

107. The apparatus according to clause 102 further comprising:

a user interface controllably coupled to the sample processing system and adapted to receive user directions wherein the controller programmably controls positioning of barrier deposition according to the user directions.

108. An apparatus comprising:

a sample processing system configured to create a Micro-chamber containing a sample on a substrate.

109. The apparatus according to clause 108 further comprising:

a reagent dispensing device configured to apply one or more reagents to the sample; and a cover handling device operable in combination with the reagent dispensing device to automate placement and removal of Micro-chamber covers on the substrate.

110. The apparatus according to clause 108 further comprising:

a cover dispenser that individually dispenses Micro-chamber covers, the dispenser being capable of dispensing covers of multiple different sizes;
a robotic head adapted to move relative to the cover dispenser and the substrate;
an effector coupled to the robotic head that programmably grips and releases Micro-chamber covers, the effector being programmable to perform multiple functions including removing a Micro-chamber cover from the cover dispenser, moving the Micro-chamber cover to a specified position, placing the Micro-chamber cover on the substrate, and removing the Micro-chamber cover from the substrate.

111. The apparatus according to clause 110 wherein:

the effector further comprises a vacuum pad that grips and releases the Micro-chamber covers.

112. The apparatus according to clause 110 wherein:

the effector further comprises an electromagnetic attachment device that grips and releases the Micro-chamber covers.

113. The apparatus according to clause 110 wherein:

the effector further comprises a mechanical robotic attachment device that grips and releases the Micro-chamber covers.

114. The apparatus according to clause 110 wherein:

the robotic head further comprises a closed or open loop motion controller.

115. The apparatus according to clause 110 further comprising:

a controller that controls the robotic head and effector;
a program code executable on the controller and configured to control automatic
removal of a Micro-chamber cover from the cover dispenser; and

a program code executable on the controller and configured to control automatic placement of a Micro-chamber cover in a manner that encloses fluid on the substrate without air bubble creation.

116. The apparatus according to clause 110 further comprising:

a controller that controls the robotic head and effector; and
a program code executable on the controller and configured to control automatic Micro-chamber cover movement and placement on a barrier laterally containing a sample to create a Micro-chamber on the substrate.

117. The apparatus according to clause 110 further comprising:

a controller that controls the robotic head and effector; and
a program code executable on the controller and configured to control automatic formation of a barrier on the substrate, Micro-chamber cover movement and placement on a non-barrier substrate to create a Micro-chamber.

118. The apparatus according to clause 110 further comprising:

a controller that controls the robotic head and effector; and
a program code executable on the controller and configured to control automatic removal of the Micro-chamber cover from the substrate.

119. The apparatus according to clause 110 further comprising:

a controller that controls the robotic head and effector;
a sealing assembly further comprising a sealant pen, a sealant reservoir coupled to the sealant pen, a sealant valve controller, and a sealant pen valve that is manipulated by the robotic head to selectively eject a pattern of sealant and seal a Micro-chamber; and
a temperature control assembly with active heating and cooling that is independently programmable for an individual substrate of a plurality of substrates.

120. The apparatus according to clause 119 further comprising:

a program code executable on the controller and configured to control automatic formation of a Micro-chamber on the substrate.

121. The apparatus according to clause119 further comprising:

a program code executable on the controller and configured to control automatic placement of a fluid micro-volume on the substrate within the Micro-chamber.

122. The apparatus according to clause 119 further comprising:

a program code executable on the controller and configured to control automatic sealing of fluid within the Micro-chamber.

123. The apparatus according to clause119 further comprising:

a program code executable on the controller and configured to control automatic heating of the substrate while preventing fluid evaporation from the Micro-chamber.

124. The apparatus according to clause 110 wherein:

the cover dispenser is stationary during operation and the robotic head is moveable.

125. The apparatus according to clause110 further comprising:

one or more Micro-chamber covers having an epoxy pattern printed on one side to prevent sticking of adjacent Micro-chamber covers in the cover dispenser.

126. The apparatus according to clause 108 further comprising:

a plurality of substrates printed with a Teflon™ pattern in a configuration that reduces incidence and magnitude of air bubbles trapped under a Micro-chamber cover.

127. The apparatus according to clause 108 further comprising:

a robotic head adapted to move relative to the substrates;
a pipette tip handling device coupled to the robotic head that handles a plurality of different sized pipette tips;
a wash head that delivers a plurality of selected bulk solutions in a plurality of controlled volumes to a sample substrate; and
a blow head that programmably dries the sample substrate.

128. The apparatus according to clause 127 further comprising:

a sealant pen adapted to programmably dispense a selected amount of sealant in a programmed pattern on the substrates, the sealant being applied to seal a Micro-chamber cover.

129. The apparatus according to clause 108 further comprising:

a stationary platform configured to hold a plurality of substrates; and
a moveable robotic head adapted to move relative to the substrates and is programmable to automatically process the substrates and manipulate the Micro-chamber covers.

130. The apparatus according to clause 108 further comprising:

a controller that controls the sample processing system and the cover handling device to automate a process of dispensing a fluid micro-volume on the substrate and covering the fluid micro-volume with a Micro-chamber cover to create a Micro-chamber on the substrate for chemical, biological, genomics, proteomics, histology, or cytology assays.

131. The apparatus according to clause 108 further comprising:

a temperature control assembly with active heating and cooling that are independently programmable for a individual substrates of a plurality of substrates; and
a controller coupled to the temperature control assembly that manages walk-away automation of the temperature control assembly and the sample processing system to automate variable temperature processing of probe micro-volumes including high temperature processing of DNA chips, protein chips, tissues, tissue micro-assays, chemical assays, biochemical assays, and biological assays, as well as Hybridization, Fluorescence In Situ Hybridization (FISH), In Situ Hybridization (ISH) assays, and other assays involving a ligand and molecular target.

132. A method of processing a sample comprising:

automatically covering a Micro-chamber on a substrate;
automatically removing a cover from the Micro-chamber;
treating a sample in the Micro-chamber with at least one selected fluid; and
automatically removing the cover from the Micro-chamber.

133. An apparatus comprising:

a sample processing system configured to mix a micro-environment within a Micro-chamber containing a sample on a substrate.

134. The apparatus according to clause 133 further comprising:

a robotic head adapted to move relative to the substrate;
a member coupled to the robotic head; and
a controller communicatively-coupled to the robotic head and adapted to manipulate the robotic head and member relative to the Micro-chamber and generate a vibration in the micro-environment.

135. The apparatus according to clause 133 further comprising:

a vibration motor positionable in the substrate vicinity; and
a controller coupled to the vibration motor and adapted to generate a vibration in the micro-environment.

136. The apparatus according to clause 133 further comprising:

a piezo-electric transducer positionable in the substrate vicinity; and
a controller coupled to the piezo-electric transducer and adapted to generate a vibration in the micro-environment.

137. The apparatus according to clause 133 further comprising:

a temperature control assembly with active heating and cooling positionable in the substrate vicinity; and
a controller coupled to the temperature control assembly and adapted to generate motion in the micro-environment by temperature cycling.

138. An apparatus comprising:

a liquid dispenser that programmably dispenses liquid to a chemical and/or biological sample on a substrate in a selected volume range including a capability to consistently dispense a selected liquid in volumes as low as 0.1 ul; and
a pipette tip handling device coupled to the liquid dispenser that aspirates and dispenses a micro-volume of the selected liquid via a pipette tip selected from among a plurality of different sized pipette tips.

139. The apparatus according to clause 138 further comprising:

a wash head coupled to the pipette tip handling device and configured to deliver a plurality of selected bulk solutions in a plurality of controlled volumes to a sample substrate; and
a blow head coupled to the pipette tip handling device and configured to programmably remove excess liquid from the sample.

140. The apparatus according to clause 138 further comprising:

a sealant pen configured to programmably dispense a selected amount of sealant in a programmed pattern on the sample substrate, the sealant being applied to seal a Micro-chamber cover.

141. The apparatus according to clause 138 further comprising:

a sealant pen configured to programmably dispense a selected sealant material to selectively form a permanent seal or a non-permanent seal.

142. The apparatus according to clause 141 wherein:

a sealant material is a polymer that forms a seal with increasing temperature and breaks the seal at a lower temperature.

143. The apparatus according to clause 138 further comprising:

a reagent head coupled to the pipette tip handling device and adapted to deliver a reagent volume to the sample substrate in a range from microliters ($\mu$l) to milliliters (ml); and
a wash head coupled to the pipette tip handling device and configured to deliver a bulk solution in a range from tens to thousands of microliters ($\mu$l).

144. The apparatus according to clause 138 further comprising:

a pipette tip handling device configured to handle a plurality of pipette tip sizes including a first size with a size range from hundreds to thousands of microliters and a second size with a size range of tenths to hundreds of microliters.

145. The apparatus according to clause 138 further comprising:

a robotic head capable of moving relative to the sample substrate; and
a sealant pen coupled to the pipette tip handling device and adapted to programmably dispense a selected amount of sealant in a programmed pattern on the sample substrate, the sealant being applied to seal a Micro-chamber cover.

146. The apparatus according to clause145 wherein:

the robotic head further comprises a closed or open loop motion controller.

147. The apparatus according to clause 138 further comprising:

a stationary platform configured to hold a plurality of substrates; and
a moveable robotic head coupled to the liquid dispenser and the pipette tip handling device, the robotic head having Proportional Integral Differential (PID) or Proportional-Integrative motion control adapted to move relative to the substrates and is programmable to automatically process the substrates and uniformly aspirate and dispense the selected liquid micro-volume.

148. The apparatus according to clause 138 further comprising:

a moving platform configured to hold a plurality of substrates; and
a moveable robotic head coupled to the liquid dispenses and the pipette tip handling device, the robotic head having Proportional Integral Differential (PID) or Proportional-Integrative motion control adapted to move relative to the substrates and is programmable to automatically process the substrates and uniformly aspirate and dispense the selected liquid micro-volume.

149. The apparatus according to clause 138 further comprising:

a controller configured to control the liquid dispenser and the pipette tip handling device;
a program code executable on the controller and configured to control aspiration of a micro-volume of a fluid or reagent probe and dispensing of the micro-volume in a region constrained by a barrier containing a sample on a substrate; and
a program code executable on the controller and configured to control aspiration of a micro-volume of a probe and dispensing of the micro-volume on a sample on a non-barrier substrate.

150. An apparatus comprising:

an automated cover handling device adapted to create a Micro-chamber on a chemical and/or biological sample on a substrate;
a temperature control assembly operable in combination with the automated cover handling device, the temperature control assembly having active heating and cooling that are independently programmable for individual substrates of a plurality of substrates; and
a sealing assembly that seals the Micro-chamber during a temperature control cycle.

151. The apparatus according to clause 150 wherein:

the automated cover handling device is adapted to automate placement and removal of Micro-chamber covers.

152. The apparatus according to clause 150 wherein:

the automated cover handling device is adapted to automate placement and removal of microscope slides.

153. The apparatus according to clause 150 further comprising:

a robotic head adapted to move relative to the substrate; and
the sealing assembly further comprising a sealant pen, a sealant reservoir coupled to the sealant pen, and a sealant pen valve that is manipulated by the robotic head to selectively eject a pattern of sealant and seal a Micro-chamber.

154. The apparatus according to clause 150 further comprising:

a controller adapted to control the automated cover handling device, the temperature control assembly, and the sealing assembly; and
the sealing assembly further comprising a sealant pen, a sealant reservoir coupled to the sealant pen, and a sealant pen valve that is manipulated by the robotic head to selectively eject a pattern of sealant and seal a Micro-chamber.

155. The apparatus according to clause 154 further comprising:

a program code executable on the controller and configured to control automatic sealing of fluid within the Micro-chamber.

156. The apparatus according to clause 154 further comprising:

a program code executable on the controller and configured to control application of a sealant to a barrier on a barrier substrate and/or to a Micro-chamber cover interface to seal the Micro-chamber.

157. The apparatus according to clause 154 further comprising:

a program code executable on the controller and adapted to control the temperature control assembly and the sealing assembly to seal a Micro-chamber at elevated temperature with reduced or eliminated evaporative fluid loss; and
a program code executable on the controller and configured to control the sealing assembly to seal a Micro-chamber.

158. The apparatus according to clause 150 further comprising:

a cover dispenser configured to individually dispense Micro-chamber covers, the dispenser being capable of processing covers of multiple different sizes;
a robotic head adapted to move relative to the cover dispenser and the substrate;
an effector on the robotic head and adapted to programmably grasp and releases Micro-chamber covers, the effector being adapted to remove a Micro-chamber cover from the cover dispenser, move the Micro-chamber cover to a substrate position, place the Micro-chamber cover on a substrate, and remove the Micro-chamber cover from the substrate;
a controller adapted to control the robotic head and effector; and
the sealing assembly further comprising a sealant pen, a sealant reservoir coupled to the sealant pen, and a sealant pen valve adapted for manipulation by the robotic head to selectively eject a pattern of sealant and seal a Micro-chamber.

159. The apparatus according to clause 158 wherein:

the robotic head further comprises a closed or open loop motion controller.

160. An apparatus comprising:

a sample handing system adapted to apply at least one selected reagent to a chemical and/or biological sample on a substrate in an automated process;
an automated cover handling device configured to operate in combination with the sample processing system to automate placement and removal of Micro-chamber covers and create a Micro-chamber on the substrate; and
a temperature control assembly coupled to the sample processing system and the automated cover handling device, the temperature control assembly being adapted to heat and actively cool individual substrates of a plurality of substrates independently and programmably during a temperature cycle.

161. The apparatus according to clause 160 further comprising:

a substrate carrier adapted for usage in combination with the temperature control assembly and having a capacity for holding an plurality of substrates in contact with a plurality of temperature control elements corresponding to the substrate plurality in the temperature control assembly.

162. The apparatus according to clause 161 further comprising:

the substrate carrier is constructed from materials that reduce or minimize thermal convection and hold a substrate secure during removal of a Micro-chamber from a substrate.

163. The apparatus according to clause 161 further comprising:

the substrate carrier comprising a carrier frame and a plurality of inserts that reduce thermal cross-talk between the substrates, the carrier frame constructed from aluminum and the inserts constructed from aluminum or thermal-insulating plastic.

164. The apparatus according to clause 161 further comprising:

the temperature control assembly including a plurality of temperature control elements, an individual temperature control element comprising a thermally-conductive temperature application top configured to make contact to a corresponding substrate.

165. The apparatus according to clause 164 wherein:

the temperature control elements are selected from among resistance heaters and heal/cool Thermo-Electric Cooler (TEC).

166. The apparatus according to clause 164 wherein:

the temperature control assembly further comprises a temperature control base coupled to a plurality of individual temperature control elements, the temperature control base being constructed from temperature and chemical resistant polymers or metals selected from a group comprising polypropylene, Kynar™, Teflon™, fluoropolymers, aluminum, and stainless steel.

167. The apparatus according to clause 166 wherein the individual temperature control elements further comprise:

a thermal-conducting metal plate;
a temperature-sensing device;
a resistive heater or thermal electric heater/cooler; and
a sealed housing that thermally, chemically, and electrically isolates the individual substrate temperature control elements.

168. The apparatus according to clause 166 further comprising:

a waste drain tray coupled into the temperature control base.

169. The apparatus according to clause 164 further comprising:

a controller coupled to the temperature control assembly that controls temperature applied to individual substrate positions of the substrate carrier according to sensor feedback.

170. The apparatus according to clause 160 further comprising:

a controller coupled to the sample processing system and the temperature control assembly; and
a program code executable on the controller comprising one or more program codes in a group consisting of:

a program code configured to execute temperature-controlled hybridization and staining simultaneously on different substrates;

a program code configured to control automatic processing of a biological and/or chemical micro-assay;

a program code configured to control automatic processing of DNA and protein microchips;

a program code configured to control automatic processing of tissue micro-assays, Fluorescence In Situ Hybridization (FISH), In Situ Hybridization (ESH), and Immunohistochemistry (IHC) samples;

a program code configured to a combination of control automatic processing of a biological and/or chemical micro-assay on a sample;

a program code configured to control automatic processing of a combination of tissue micro-assays, Fluorescence In Situ Hybridization (FISH), In Situ Hybridization (ISH), and Immunohistochemistry (IHC) samples on a sample;

a program code configured to automatically control user-determined substrate temperature and incubation times;

a program code configured to automatically control over-temperature protection and safety control;

a program code configured to control active heating and cooling of the individual substrates to a selected temperature set-point; and

a program code configured to control automatic active heating and cooling of a Micro-chamber to a high temperature and hold the temperature for a selected time without loss of a significant quantity of fluid.

171. An apparatus comprising:

a fluid dispenser operative in a system for processing chemical and/or biological samples, the fluid dispenser being adapted to dispense one or more selected fluids to a selected sample; and

a fluid level detector comprising a combined vacuum detector and pressure detector.

172. The apparatus according to clause 171 further comprising:

a controller coupled to the fluid level detector and adapted to selectively operate the vacuum detector for measuring fluid level for relatively large volume, low viscosity fluids.

173. The apparatus according to clause 171 further comprising:

a metering pump that is cycled to create a vacuum source;

a vacuum switch that detects a change in pressure; and

a controller adapted to receive a signal indicative of the change in pressure.

174. The apparatus according to clause 173 further comprising:

a robotic handler adapted to manipulate a pipette including a pipette tip;

the controller being adapted to lower the pipette into a fluid container, receive a signal from the vacuum switch on detection of the pressure change when the pipette tip touches the fluid surface in the container, save pipette position information at the pressure change, and determine a distance to move the pipette to aspirate a selected fluid volume.

175. The apparatus according to clause 171 further comprising:

a controller coupled to the fluid level detector and adapted to selectively operate the pressure detector for measuring fluid level for relatively low volume, high viscosity fluids.

176. The apparatus according to clause 171 further comprising:

a metering pump;

a pressure switch that determines a positive pressure; and

a controller adapted to receive a signal indicative of the pressure.

177. The apparatus according to clause 176 further comprising:

a robotic handler adapted to manipulate a pipette including a pipette tip;

the controller being adapted to lower the pipette into a fluid container, receive a signal from the pressure switch on detection of a pressure change when the pipette tip nears the fluid surface in the container, save pipette position information at the pressure change, and determine a distance to move the pipette to aspirate a selected fluid volume.

178. The apparatus according to clause 171 further comprising:

a sample processing system adapted to dispense at least one reagent fluid to a chemical and/or biological sample; and
one or more reagent/probe containers, the fluid dispenser and fluid level detector being adapted to determine fluid level in the one or more reagent/probe containers.

179. The apparatus according to clause 171 further comprising:

a controller coupled to the fluid level detector and adapted to selectively operate the vacuum detector and the pressure detector to reduce or eliminate bubbles in the liquid.

180. An apparatus comprising:

a sample processing system adapted to apply at least one selected reagent to a chemical and/or biological sample in an automated process, the sample processing system further comprising one or more reagent/probe containers;
a vacuum and pressure source coupled to the sample processing system and operative for dispensing fluids;
a vacuum and pressure sensor coupled to the sample processing system and operative for measuring a condition of the reagent/probe containers; and
a fluid level detector coupled to the sample processing system, the vacuum and pressure source, and the vacuum and pressure sensor, the fluid level detection device being adapted to detect fluid level in the reagent/probe containers selectively based on either vacuum or pressure changes.

181. The apparatus according to clause 180 further comprising:

a controller coupled to the fluid level detector and adapted to selectively operate the vacuum and pressure sensor to measure vacuum to determine fluid level for relatively large volume, low viscosity fluids.

182. The apparatus according to clause 180 further comprising:

a controller coupled to the fluid level detector and adapted to selectively operate the vacuum and pressure sensor to measure pressure to determine fluid level for relatively low volume, high viscosity fluids.

183. The apparatus according to clause 180 further comprising:

a controller coupled to the automated fluid level detection device;
a program code executable on the controller and configured to control automatic sensing of reagent fluid level; and
a program code executable on the controller and configured to control fluid level in the reagent/probe containers to reduce or eliminate bubbles in the reagent.

184. An apparatus comprising:

a sample processing system configured to apply at least one selected reagent to a chemical and/or biological sample on a substrate in an automated process; and
a waste separation system coupled to the sample processing system and configured to divert effluent waste from the sample processing system into a plurality of separate parts under program control.

185. The apparatus according to clause 184 further comprising:

a substrate carrier;
a waste drain tray coupled to the substrate carrier and having an outlet;
a multiple-way valve coupled to the waste drain tray outlet; and

a pump coupled to the waste drain tray outlet and being programmably controlled to controllably separate the effluent.

186. The apparatus according to clause 184 further comprising:

a controller coupled to the sample processing system, the multiple-way valve, and the pump, the controller being adapted to programmably automate application of the at least one reagent and separation of effluent in a combined operation.

187. The apparatus according to clause 184 further comprising:

a controller coupled to the sample processing system, the multiple-way valve, and the pump, the controller being adapted to programmably automate application of the at least one reagent and separation of toxic from non-toxic waste in a combined operation.

188. The apparatus according to clause 184 further comprising:

a controller coupled to the sample processing system, the multiple-way valve, and the pump, the controller being adapted to programmably automate application of the at least one reagent and separation of a plurality of different waste effluents in a combined operation.

189. The apparatus according to clause 184 further comprising:

a controller coupled to the sample processing system, the multiple-way valve, and the pump, the controller being adapted to programmably automate application of the at least one reagent and diversion of a plurality of different waste effluents to different waste receptacles in a combined operation.

190. The apparatus according to clause 184 further comprising:

a substrate carrier further comprising a temperature control base coupled to a plurality of individual substrate temperature control assemblies, and a waste drain tray coupled to the temperature control base, the waste drain tray having an outlet; and
a multiple-way valve coupled to the waste drain tray outlet, the multiple-way valve being programmably controlled to controllably separate the effluent by gravity flow.

191. A system comprising:

a controller adapted to connect to a network, communicate with a plurality of sample processing systems, and collect sample processing information from the plurality of sample processing systems.

192. The system according to clause 191 further comprising:

the controller adapted to share information among the plurality of sample processing systems.

193. The system according to clause 191 further comprising:

the controller adapted to generate information logs and/or statistics relating to operations of one or more of the sample processing systems.

194. The system according to clause 191 further comprising:

the controller adapted to track reagent usage including tracking of reagent volume usage per reagent container.

195. The system according to clause 194 further comprising:

the controller adapted to track reagent usage independent of device or component usage the reagent.

196. A system comprising:

a network adapted to communicate with a plurality of sample processing systems and collect sample processing information from the plurality of sample processing systems.

197. The system according to clause 196 further comprising:

the network adapted to share information among the plurality of sample processing systems.

198. The system according to clause 196 further comprising:

the network adapted to track reagent usage including tracking of reagent volume usage per reagent container.

199. The system according to clause 198 further comprising:

the network adapted to track reagent usage independent of device or component usage the reagent.

200. A method of operating on a network comprising:

communicating with a plurality of sample processing systems; and
collecting sample processing information from the plurality of sample processing systems.

201. The method according to clause 200 further comprising:

sharing information among the plurality of sample processing systems.

202. The method according to clause 200 further comprising:

tracking reagent usage including tracking of reagent volume usage per reagent container.

203. The method according to clause 202 further comprising:

tracking reagent usage independent of device or component usage the reagent.

## BRIEF DESCRIPTION OF THE DRAWINGS

Embodiments of the invention relating to both structure and method of operation, may best be understood by referring to the following description and accompanying drawings whereby:

**FIGUREs 1A-1D** are perspective pictorial diagrams illustrating various embodiments of a substrate, such as a slide, forming part of a MicroChamber;

**FIGUREs 2A-2C** are perspective pictorial diagrams illustrating various embodiments of a MicroChamber including a MieroChamber cover;

**FIGURE 3** is a perspective pictorial diagram that shows an embodiment of a substrate labeled using a radio frequency identifier;

**FIGURE 4** is a perspective pictorial diagram depicting an embodiment of a substrate with a barrier formed by a sample processing system;

**FIGUREs 5A** and **5B** are perspective pictorial diagrams showing an embodiment of MicroChamber with a cover that includes a vesicle for dispensing liquid to the microenvironment within the MicroChamber;

**FIGURES 6A-6C** show multiple views of an embodiment of a sample processing system that is adapted to concurrently and individually control processing of a plurality of samples;

**FIGURE 7** shows an embodiment of a sample processing system including a humidity controller;

**FIGUREs 8A-8D** show embodiments of various devices that can be used as the cover handling device of FIGUREs 6A-6C;

**FIGURE 9A** shows an embodiment of a sealing assembly as an attachment to the robotic device of **FIGUREs 6A-6C**;.

**FIGURE 9B** shows an embodiment of the sealant assembly configured independently of the robotic device of **FIGUREs 6A-6C;.**

**FIGUREs 10A-10E** shows an embodiment of reagent dispensing device of the robotic device of **FIGUREs 6A-6C**;.

**FIGUREs 11A -11G** are pictorial views showing various embodiments of temperature control assemblies that enable independent heating and cooling of multiple temperature control elements;

**FIGURE 12** shows an embodiment of a platform comprising a carrier frame and a plurality of inserts that reduce thermal cross-talk between the substrates of **FIGUREs 6A-6C;**

**FIGURE 13** shows an embodiment of a fluid handler for usage in the sample processing system of **FIGUREs 6A-6C;** and

FIGURE 14 shows an embodiment of a waste handling system that can be used in the sample processing system of **FIGUREs** 6A-6C.

## DETAILED DESCRIPTION

To solve the challenges of the post-genomic era and to accelerate clinical diagnostics and drug discovery development, embodiments of a sample processing system that automates processing of a sample, such as a chemical and/or biological sample, are disclosed. The sample processing system enables total walk-away, industrial scale, streamlined, and standardized sample processing and testing for multiple samples simultaneously in applications such as DNA microarrays, protein microarrays, Fluorescence In Situ Hybridization (FISH), In Situ Hybridization (ISH) assays, Immunohistochemistry (IHC) samples, staining, and image analysis.

Sample processing systems disclosed herein can perform functions such as (a) individual slide temperature control, (b) dispensing a wide range of reagent volumes from nanoliters (vl) to multiple milliliters (ml), (c) microtiter plate capabilities for small volume reagents, (d) automated placement and removal of reservoir covers and cover slips, (e) walk-away automation with minimal user intervention, (f) barcode and Radio Frequency Identification (RFID) tracking for protocols, reagents, and slides among multiple sample processing systems, (g) independent, environmentally-controlled processing for multiple slides or substrates, (h) reagent temperature control, (i) over-temperature protection and control, (j) liquid level sensing in a reagent/probe container, (k) usage of disposable pipette tips in a range of sizes for dispensing variable quantities of substances, and (l) centralized user interface to one or more sample processing systems. The sample processing systems can also provide capability to process deoxyribonucleic acid (DNA) and protein microchips, and the capability to process multiple samples using different protocols simultaneously, such as tissue arrays, Fluorescence In Situ Hybridization (FISH), In Situ Hybridization (ISH) assays, and Immunohistochemistry (IHC) samples.

The sample processing system can also be configured to create a MicroChamber in which any suitable process can be performed, such as chemical, biological, genomics, proteomics, histology, and/or cytology assays below, at, and/or above room temperature and humidity, thereby creating an enclosed microenvironment for sample processing.

Other features of the system enable automatically separating toxic waste from non-toxic waste; loading and unloading slides; and prioritizing slide processing.

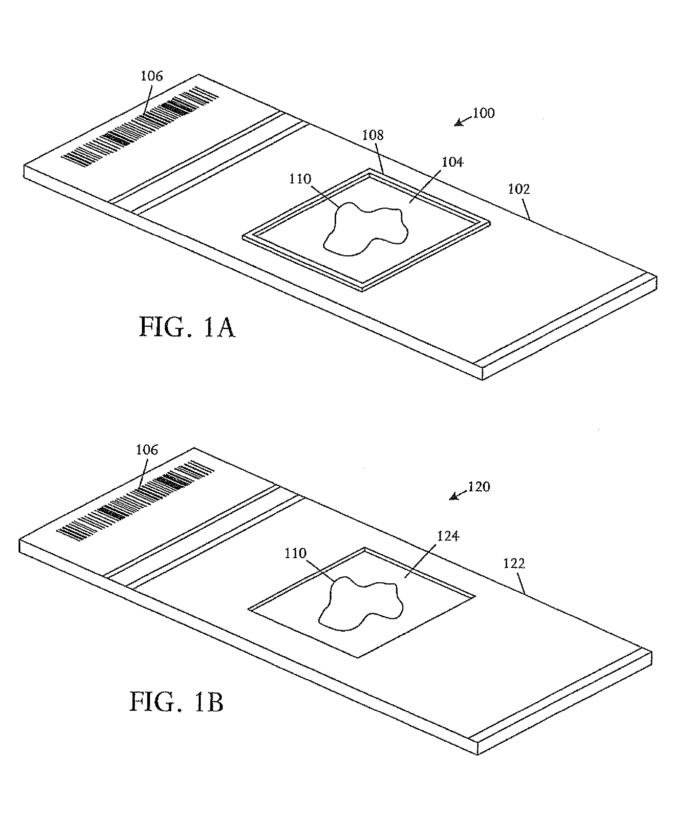

Referring to **FIGURE 1A,** an embodiment of a MicroChamber **100** is shown that includes a substrate **102,** a reservoir **104** formed on the substrate **102** for receiving one or more reagents and/or a sample **110,** such as a chemical and/or biological sample, and a barcode **106.** An encoder, such as barcode **106** can be formed on the substrate **102** to encode information describing at least one characteristic of the substrate **102** and/or the reservoir **104.** Note that other suitable encoder devices can be used in addition to, or instead of, the barcode **106.**

A sample processing system can be configured to scan barcodes **106** to identify various characteristics of MicroChamber **100.** In some embodiments, the barcode **106** encodes any suitable information such as reservoir volume, reservoir size, reservoir shape, reservoir depth, number of reservoirs, substrate material, substrate electrical characteristics, substrate color, presence and/or properties of components attached to the substrate, and/or sample type, among other items.

The reservoir **104** can be constructed using a barrier **108** formed on a substrate **102.** The barrier **108** has a shape, size, and height selected to contain a specified area or volume. In some embodiments, the barrier **108** can be formed on a substrate or slide that holds a pre-applied sample **110,** however, the barrier **108** can be formed on the substrate **102** before or after application of the sample. Any suitable material or substance can be used to form the barrier **108,** such as epoxy, a wax film (e.g., Parafilm™), Teflon™, and/or oil. The barrier **108** may be applied using any suitable method, such as adhesive tape, annealing, ink (e.g., Teflon™ ink), paint, and/or deposition. In some embodiments, the barrier **108** can be constructed from a hydrophobic material or substance to contain hydrous as well as anhydrous substance(s) within the reservoir **104.** In some embodiments, the barcode **106** may include information regarding the size of the barrier **108,** and/or a sealing device or sealing agent that is used to create the barrier **108** around the reservoir **104.**

The reservoir **102** and barrier **108** can be configured to reduce or even eliminate air bubbles in reservoir **102** when the reservoir **102** is covered to form an enclosed microenvironment. Such a configuration can also reduce the volume of reagent used in a process or application, as well as evenly distribute substances in reservoir **102.**

Referring to **FIGURE 1B,** another embodiment of a MicroChamber **120** is shown in which a barrier **108** around the reservoir **124** is formed as a depression in the substrate **122.** The shape, size, and depth of the reservoir **124** can be selected to contain a specified volume. Programmed control of barrier deposition speed can be implemented to enable deposition of different materials with various flow rates and solidification times and determine barrier thickness based on the viscosity of the substance used to create the barrier **108.**

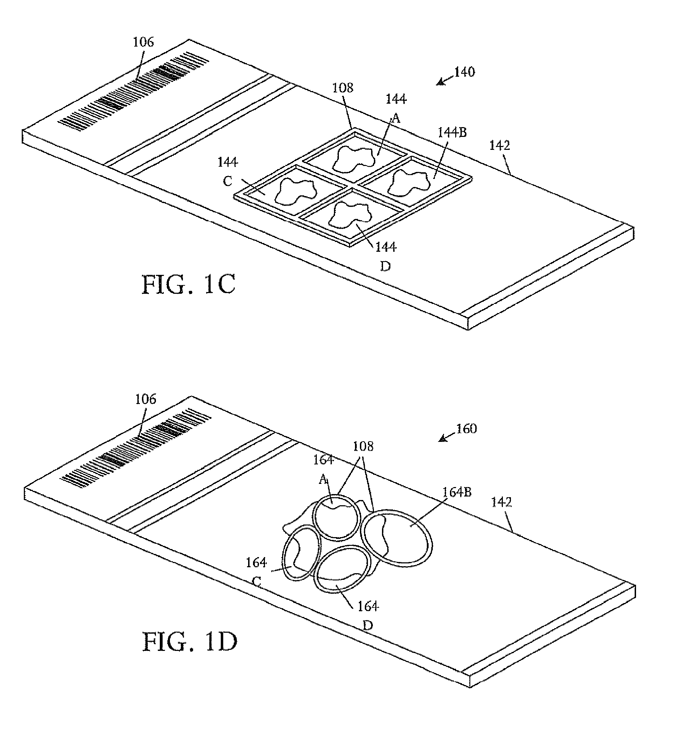

Referring to **FIGURE 1C,** an embodiment of another MicroChamber **140** is shown that includes a plurality of reservoirs **144A-D** formed on the substrate **142** to accommodate a sample, such as a chemical and/or biological sample. The reservoirs **144A-D** are depicted with the same shape, size, and depth and containing the same volume. In other embodiments, for example as shown in **FIGURE 1D**, reservoirs **164A-F** may vary in shape, size, depth, and/or volume.

In various embodiments, the substrate **102, 122, 142, 162** may be formed from glass, for example a glass dish, slide, microscope slide, bowl, or the like. Other suitable substrate materials can be used, such as thermally-conductive materials, electrically conductive or non-conductive materials, piezo-electric materials, silicon materials, polymer materials, and others.

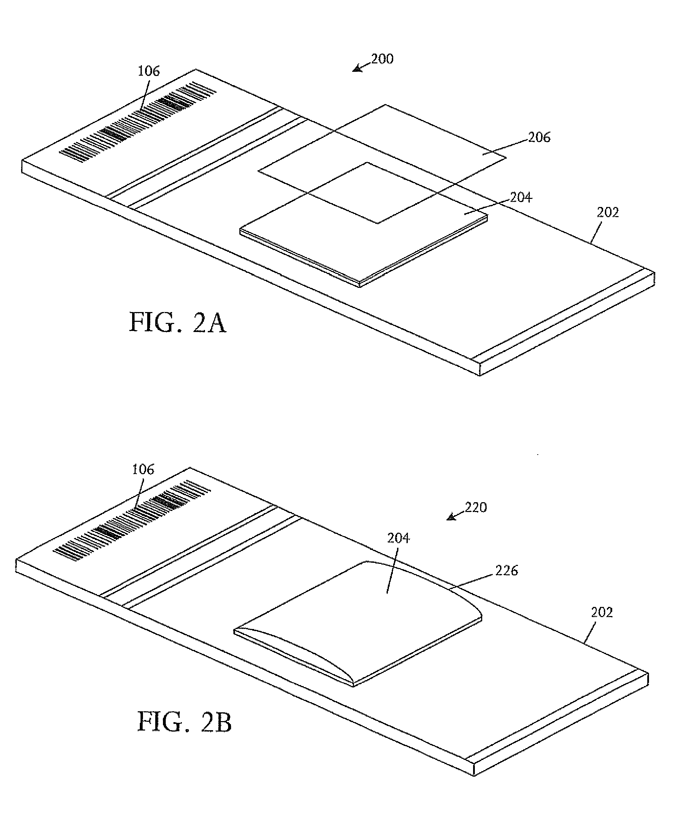

Referring to **FIGURE 2A,** an embodiment of a MicroChamber **200** is shown including a substrate **202,** a reservoir **204,** and a cover **206** that can be positioned on substrate **202** to enclose reservoir **204** and form a microenvironment for processing the sample. The cover **206** can be any suitable device, such as a cover slip, with a size and shape configured to cover the periphery of reservoir **204** and/or the barrier **108 (FIGURE 1A).** In another embodiment of a MicroChamber **220,** for example as shown in **FIGURE 2B,** the cover **226** can be a cap that is configured to enclose the reservoir **204.** In various embodiments, the cover **206, 226** is constructed from any suitable material, such as glass, plastic, hard plastic, polymer, and/or one or more layers, such as layers formed from solidified or dried layers of a substance.

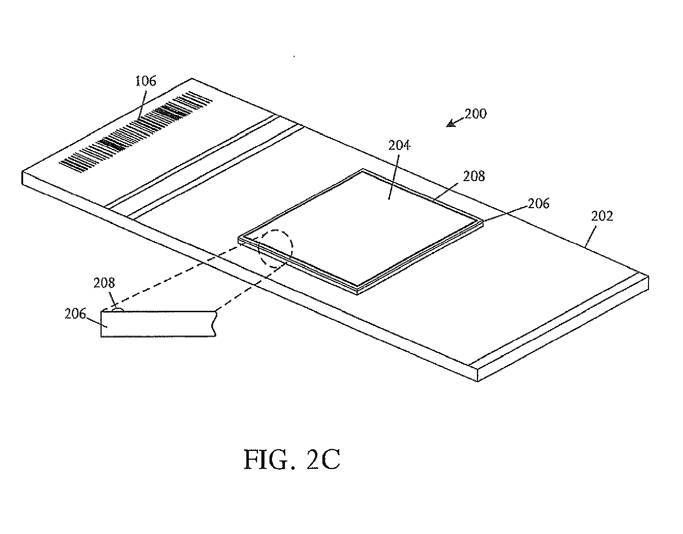

**FIGURE 2C** shows an embodiment of the MicroChamber **200** that further includes a coating **208** formed on a cover **206,** the figure also depicting an insert showing a magnified view of the cover **206** with coating **208** around the edge of cover **206.** The coating **208** can be used to help preventing adjacent covers **206** from adhering to one another, and can be any suitable material or substance, such as paint, metal, powder, lamination, foil, and the like. The coating **208** can be applied in any suitable location on cover **206** and can have properties that allow the position and orientation of cover **206** to be detected automatically. For example, the coating **208** can have conductive properties that allow the coating **208** to be detected electronically, and/or optical properties that allow the coating to be detected with optical sensors. In some embodiments, the position and orientation of cover **206** can be determined when the coating **208** is applied in a recognizable pattern around one or more portions of the periphery or other suitable location on cover **208.** The coating **208** can also be configured to form at least a portion of barrier **108 (FIGURE 1A)** in some embodiments. A signal processing system (not shown) can be configured to determine the position and orientation of the cover **206** based on sensor signals. The sample processing system can use the position and orientation information to locate and position the cover(s) **206** as desired.

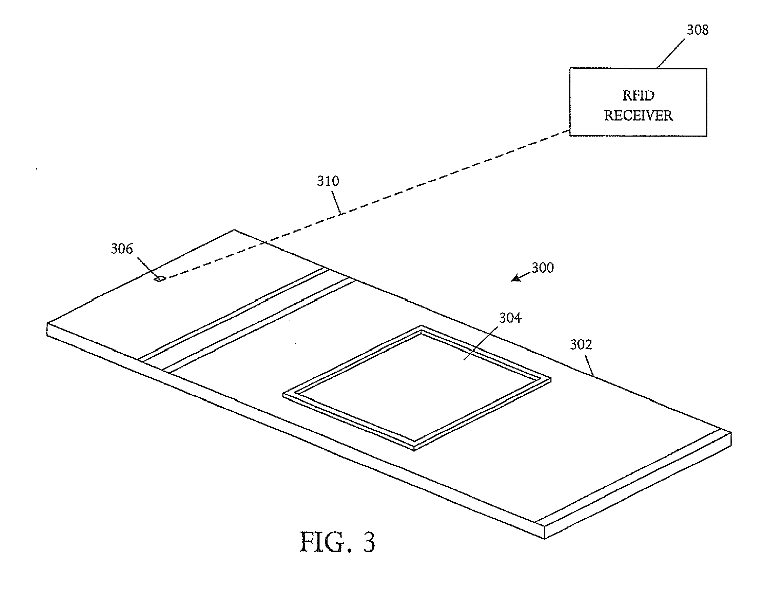

Referring to **FIGURE 3,** an embodiment of another MicroChamber **300** is shown that includes a substrate **302,** a reservoir **304** formed on the substrate **302** that can receive a sample, such as a chemical and/or biological sample, and a radio frequency identification tag (RFID) **306.** The term RFID describes the use of radio frequency signals to provide information regarding the identity, location, and other characterizing information about MicroChamber **300.** The RFID tag **306** coupled to the substrate **302** can transmit RF signals **310** that include information describing at least one characteristic of the substrate **302** and/or reservoir **304.** For example, the RFID tag **306** may encode at least one information item such as a unique identifier for the MicroChamber **300,** reservoir volume, reservoir size, reservoir shape, reservoir depth, number of reservoirs, substrate material, substrate electrical characteristics, substrate color, characteristics of the barrier **108 (FIGURE 1A),** and the presence and/or properties of components and samples attached to and mounted on the substrate **302.** An RFID receiver **308** in the transmitting range of RFID tag **306** can detect signals **310** transmitted by RFID tag **306** and use the information in a processing system that tracks any suitable aspects of MicroChamber **300,** such as inventory, configuration, location, and current state of usage af the MicroChamber **300.**

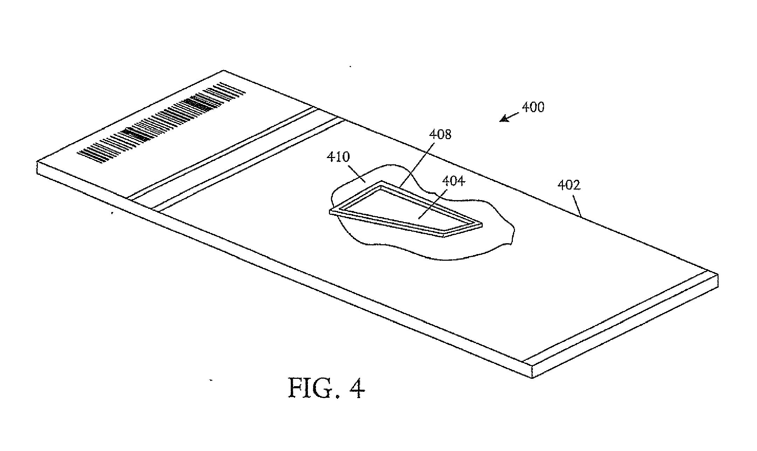

Referring to **FIGURE 4,** a MicroChamber **400** is shown that comprises a substrate **402,** a barrier **408,** and a reservoir **404** enclosed by the barrier **408.** A sample **410** can be applied to the substrate **402** before the substrate **402** is inserted or placed into the sample processing system for handling. The barrier **408** can be constructed to a selected shape, size, and height selected to contain a specified volume. As a result, a portion of a pre-applied sample **410** may lie outside barrier **408.** Alternatively, the barrier **408** can be formed on the substrate **402** around the pre-applied chemical and/or biological sample **410** with the barrier position, size, shape, thickness and/or volume controlled by the sample processing system. The characteristics of the barrier **408** can also be controlled by the user according to directions and commands received via a user interface to form one or more reservoirs corresponding to one or more regions of interest in the chemical and/or biological sample.

The sample processing system can be controlled to produce a single barrier **408** and single reservoir **404,** or multiple barriers and corresponding multiple reservoirs having the same or different shapes and sizes. In some embodiments, double barriers **408** are used. Sealant can be placed in between the double barrier or outside or inside of the barriers. A sealing device or sealing agent can be used between the double barriers **408,** and/or inside or outside of the barriers **408.**

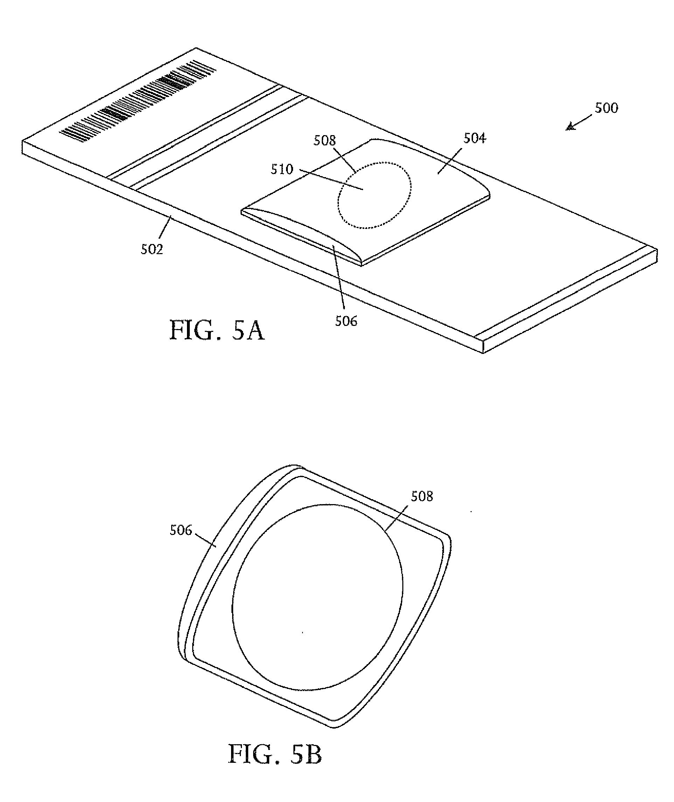

Referring to **FIGURE 5A,** an embodiment of a MicroChamber **500** is shown comprising a substrate **502,** a reservoir **504** formed on the substrate **502,** a cover **506** that can be secured to the substrate **502** to contain the reservoir **504,** and a vesicle **508.** The vesicle **508** can be attached to or integrally formed with the cover **506** and contains a substance, such as a reagent.

**FIGURE 5B** shows a view of the vesicle **508** in the cover **506,** with the cover **506** being adapted to contain substances. The vesicle **508** is constructed to securely contain a specified volume of substance such as a reagent until application of the substance to a sample is desired. The vesicle **508** can be further adapted to be opened or breached at a suitable time to enable application of the substance in the vesicle **508** to the sample. For example, in some embodiments, the vesicle **508** can be dissolvable when contact is made with substance(s) in the reservoir **504.** The vesicle **508** may be configured to rupture upon contact with the substrate **502.** In another example, a sharp surface may be formed on the substrate or the cover that cuts a portion of the vesicle **508** when the cover **506** is placed on the substrate **502.** In some embodiments, the vesicle **508** can be constructed of a material that dissolves upon chemically reacting with substance(s) in the reservoir **504,** upon reaching a specified temperature, or other suitable method. In other embodiments, the cover **506** can be coated with a substance that combines with substances in the reservoir **504** when the cover **506** is positioned on the reservoir **504.**

The covers **206, 226, 506** can have different shapes, sizes, color, opacity, or other specified characteristics, depending on the requirements for the sample processing system. For example, some processes may require exposing the contents of the reservoir **204, 304, 404, 504** to light having a specified wavelength. Some of the covers can be configured as a filter to allow light of the specified wavelength to reach the contents of the reservoir **204, 304, 404, 504.** Further, the covers **206, 226, 506** can be changed during a process, for example, when a cover having a particular characteristic is required during a particular phase of the process but is not suitable for other phases of the process. The covers **206, 226, 506** can be any suitable material, and can further be laminated, disposable, reusable, washable, and/or dryable.

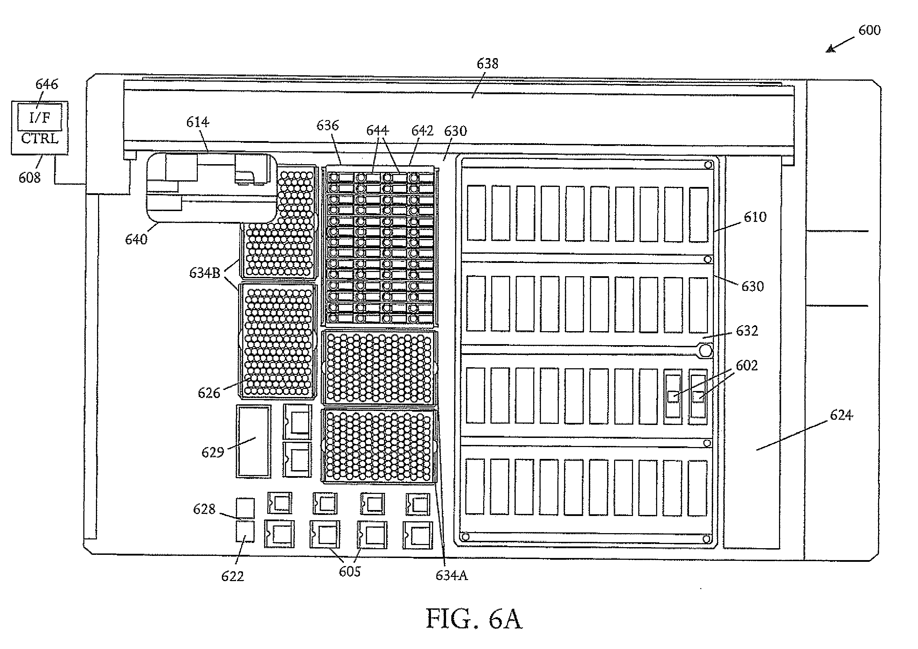

Referring to **FIGURES 6A-6C,** multiple views of a sample processing system 600 that is adapted to concurrently and individually control processing of a plurality of samples is shown. The illustrative sample processing system **600** is a self-contained, automated system with cover placement and removal capabilities, precision aspirating and dispensing of reagents, and individual temperature control for MicroChambers **602.**

In some embodiments, sample processing system **600** includes a platform **630** and rack **642** that can be held by the platform **630** or coupled to the platform **630** and adapted to hold multiple reagent containers **644.** The rack **642** can also be configured with one or more individually controllable heating elements to maintain the reagents at different selected temperatures. The sample processing system **600** can also be configured to independently maintain a plurality of MicroChambers, such as , MicroChamber **200** in **FIGURE 2C,** at different environmental conditions, such as different temperature, light, and/or humidity levels.

In some embodiments, the robotic device **640** is mounted on a movable arm **614** that can be positioned in one, two, and/or three dimensions relative to the platform **630.** The robotic device **640** can be configured to accept different types of attachments to perform various different operations and functions, such as gripping and releasing covers; positioning and removing a cover from a reservoir, such as cover **206** and reservoir **204** in **FIGURE 2A;** loading and dispensing substances; loading and dispensing sealant to create a barrier, such as barrier **108** in **FIGURE 1A;** mixing MicroChamber contents; washing a MicroChamber **602;** and drying a MicroChamber **602,** among others.

In some embodiments, the robotic device **640** includes a cover handling device **606** adapted to dispense covers of one or more sizes on reservoirs to form the MicroChambers **602.** The cover handling device **606** can be adapted to retrieve loose covers from cover storage boxes 605 and/or other suitable location in or around the sample processing system **600.** The robotic head **640** can further include a metering pump, a vacuum pump, cable train and printed circuit board containing components and devices for controlling the robotic head **640.** The robotic head **640** can also incorporate a Lee Pump, a vacuum pump, optical sensors, coil latch solenoid, SMI horizontal slide, SMC vacuum switch, valves, and an Igus e-chain.

The cover storage box **605** can enable covers to be dispensed one at a time. The cover storage box **605** can be refillable and constructed from aluminum, stainless steel, plastic, or other suitable material. In some embodiments, the cover storage box **605** can be spring-loaded or otherwise configured to facilitate handling of the covers.

In further embodiments, movable covers can be formed with or attached to rack **642** adjacent one or more of the substrates. The covers can be positioned over and removed from the reservoirs with independently controlled actuators or by suitably configured robotic device(s) **640.** The robotic device **640** or other suitable mechanism can be configured to wash and/or dry the movable covers before and/or after processing a sample. The covers may be replaced with the same or different type of cover, and may be embodied using any suitable form factor such as side-hinges, accordion shape, sliding, and/or dispensable tape.

The sample processing system **600** can be configured with one or more sensors to detect the position and orientation of the covers on the MicroChambers **602** or other locations in the sample processing system **600.** In some embodiments, one or more of the sensors can be located on or in the movable arm **614** and/or robotic device **640.** The sensors can also be located in a stationary position, in addition to, or instead of, being co-located with the movable arm **614** and/or robotic device **640.**

In some embodiments, the sample processing system **600** can include a substance dispensing device **604** that is adapted to dispense one or more substances, such as a reagent, in the MicroChambers **602.** The cover handling device **606** can operate in combination with the substance dispensing device **604** to automate placement and removal of the covers over the reservoirs at the appropriate time during the process.

A controller **608** can be included in the sample processing system **600** to execute logic instructions that control operations and functionality of components in the sample processing system **600,** such as the substance dispensing device **604** and the cover handling device **606.** The controller **608** can also be adapted to operate components in the sample processing system **600** to control the microenvironment in MicroChambers **602.** Programmed logic instructions associated with particular protocols and processes can specify actions to be taken at particular times such as placing a cover on a MicroChamber **602,** removing a cover from the MicroChamber **602,** heating or cooling a reagent, dispensing a specified reagent to the MicroChamber **602;** heating or cooling the MicroChamber **602,** and/or washing the Micro-Chamber **602** and/or cover, among others. For example, a particular process can be associated with a particular Micro-Chamber **602** or group of MicroChambers **602** via a user interface. The process can specify dispensing a first reagent to a reservoir containing a sample, placing and sealing a cover on the reservoir to form a MicroChamber **602,** removing the cover from the MicroChamber **602,** washing the reagent from the MicroChamber **602,** drying the MicroChamber **602,** dispensing a second selected reagent to the MicroChamber **602,** again covering the MicroChamber **602,** and selectively repeating the various actions.

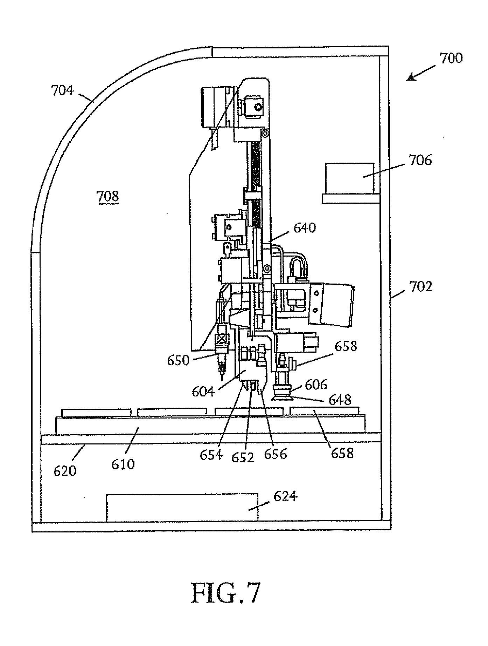

Referring to **FIGURE 7,** an embodiment of a sample processing system **700** including a humidity controller **706** is shown to control humidity in the internal cabinet environment enclosed by framework **702** and cabinet **704.** The humidity controller **706** can operate independently; or be controlled by the controller **608** or other suitable control device in combination with operation of other components in the sample processing system **600.** Typically, humidity in the sample processing system **600** can be adjusted to a level that helps prevent evaporation of contents in the MicroChambers **602.** However, in some instances humidity can be controlled for a specimen(s) in a particular MicroChamber or group of MicroChambers **602.** For example, the controller **608** can adjust humidity within the system MicroChamber **708** prior to and during exposure of the contents of one or more of the MicroChambers **602** when a cover is not in place.

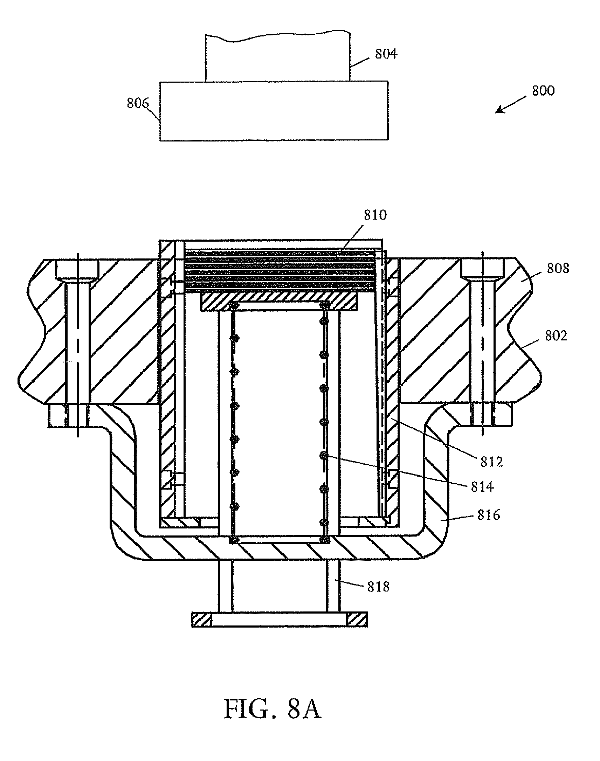

Referring to **FIGUREs 8A-8D,** embodiments of various devices that can be used as the cover handling device **606** of **FIGUREs 6A-6C** are shown. An effector **806** is coupled to a robotic head **804.** One or more dispensers **802** can dispense covers of one or more different sizes or other characteristics. The robotic head **804** is adapted to move to the vicinity of the dispenser **802** to allow the effector **806** to retrieve a cover from the dispenser **802.** The effector **806** can be operated to perform multiple functions including placing and removing covers from a substrate, such as substrate **202** in **FIGURE 2A.**

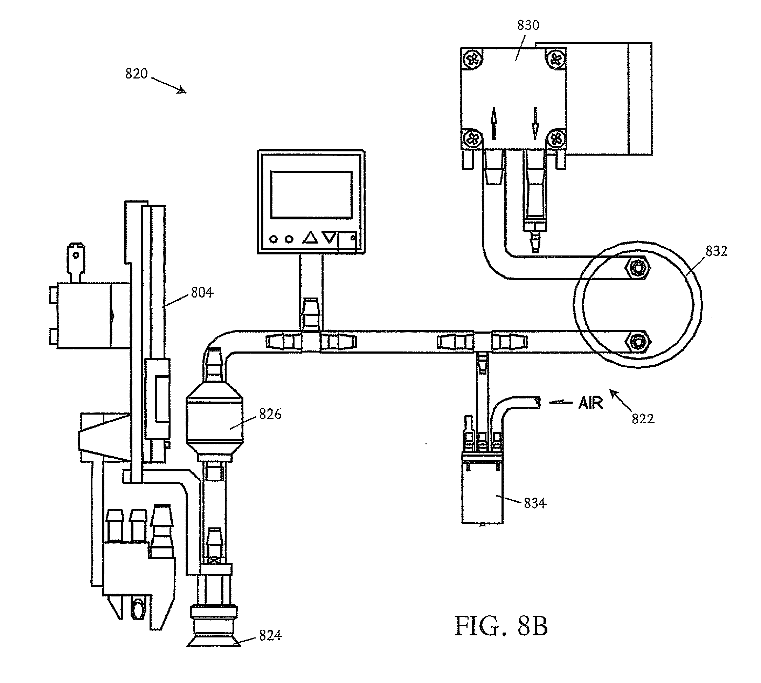

**FIGURE 8B** shows an embodiment of cover handling system **820** that includes a vacuum system **822** including a vacuum pad effector **824** that grips and releases the covers. The vacuum system **822** can include a water separator **826,** a vacuum sensor **828,** a vacuum pump **830,** a vacuum buffer **832,** and/or an air valve **834.** The vacuum sensor **828** can be configured to supply signals to controller **608 (FIGURE 6A)** to control operation of the cover handling system **820.**

When vacuum sensor **828** indicates increased pressure, logic in controller **608** assumes that a cover is obstructing an opening in effector **824** through which vacuum pressure is exerted by the vacuum pump **830.** After a cover is placed in position, the vacuum pump **830** is turned off and the air valve **834** opens, enabling positive air pressure to push the cover off the vacuum pad effector **824.** The operation prevents the cover from adhering to the vacuum pad effector **824.**

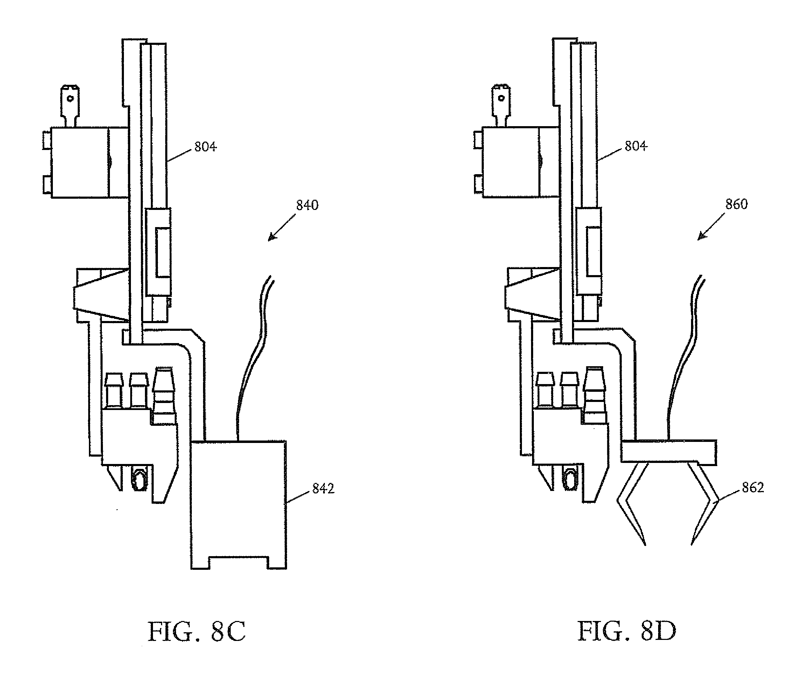

**FIGURE 8C** shows an embodiment with an electromagnetic effector **840** further comprising an electromagnetic attachment device **842** that grips and releases the covers. In such embodiments, covers are configured with one or more magnetic portions, For example, the cover may be configured with a magnetic paint or coating, chemical coating, a conductive material, foil, or other suitable material. The material can be embossed or otherwise configured to prevent covers from adhering. The electromagnetic attachment device **842** can be operated to generate positive and negative electrical fields that attract and repel the magnetic material on the covers.

**FIGURE 8D** illustrates an embodiment with an effector **860** further comprising a mechanical gripper device **862** that grips and releases the covers. The gripper device **862** can be padded, coated with rubber, or other suitable substance to facilitate handling of the covers.

In the various embodiments, the controller **608** controls operation of the robotic head **804** and the effectors **806, 824, 842, 862.** A program code executed by the controller **608** is adapted to control removal of a cover from the substrate without disturbing the sample or substrate. In some embodiments, the effector **806, 824, 842, 862** and the robotic head **804** may be configured to move independently of one another. Note that other suitable devices can be utilized, in addition to, or instead of effectors **806, 824, 842, 862.**

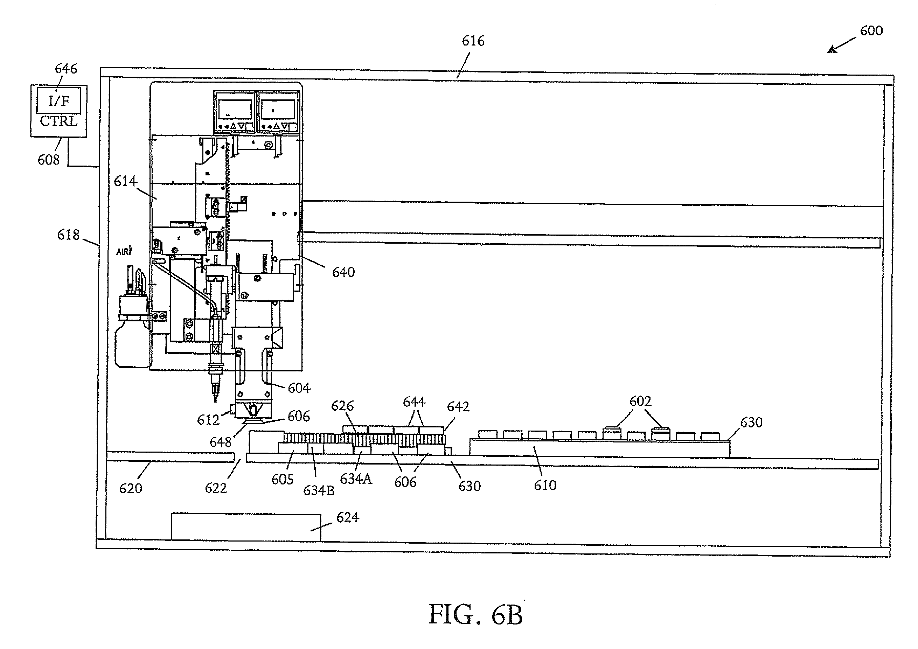

Referring to **FIGUREs 6A** and **6B,** in some embodiments, a pipette tip **626** can be used as the oil pen and grasped by the pipette tip adapter **652.** The pipette tip **626** can be dipped in a reservoir of sealing substance, such as oil, to pick up a precise amount of sealing substance. The sealing substance can be dispensed in any programmed pattern by moving the robotic device **640.** The pipette tip **626** can be discarded after usage.

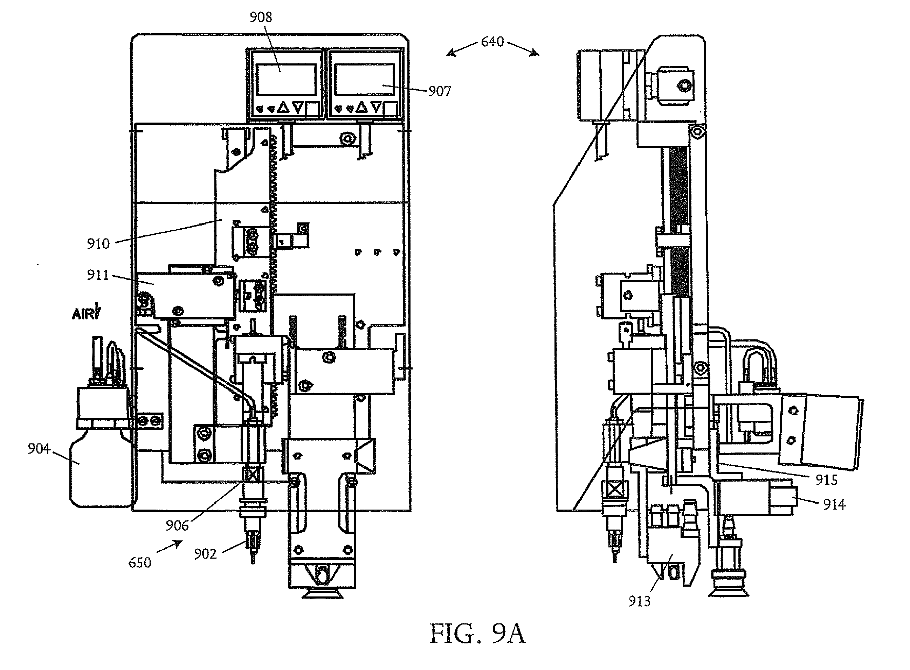

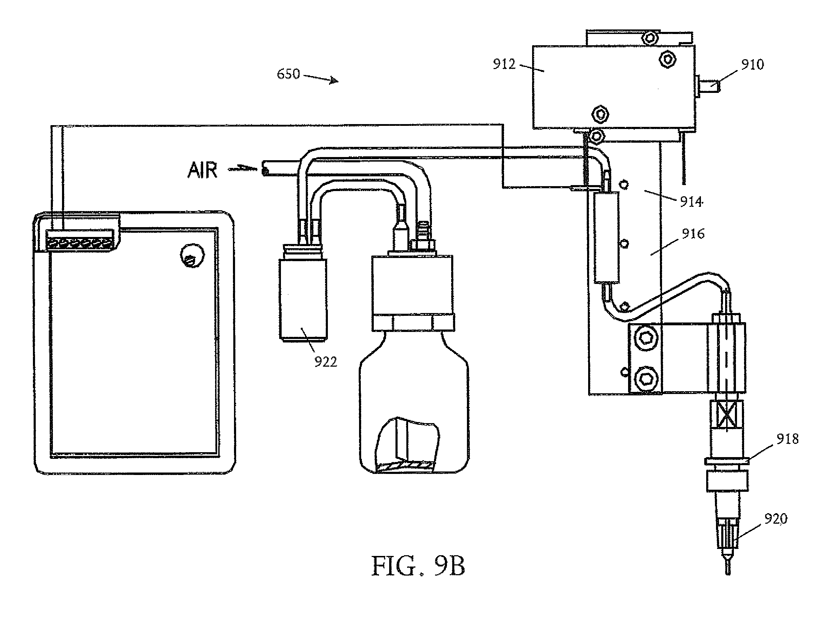

In other embodiments, the sample processing system **600** may include a sealing assembly **650.** Referring to **FIGUREs 9A** and **9B, FIGURE 9A** illustrates the sealing assembly **650** as an attachment to the robotic device **640.** **FIGURE 9B** shows an embodiment of the sealant assembly **650** configured independently of the robotic device **640.** Sealing assembly **650** can include a sealant pen **902,** a sealant reservoir **904** coupled to the sealant pen **902,** a sealant valve controller **908,** and a sealant pen valve **906** that can be operated to selectively eject a pattern of sealant to form a barrier such as barrier **108 (FIGURE 1A)** around a reservoir **104,** and/or to seal a cover over the reservoir. The sealant pen **902** can include an application attachment such as a syringe or needle. **The** sealant reservoir **904** contains a suitable sealing material such as a sealing oil, wax, polymer, or suitable substances. The sealant valve controller **908** enables the valve **906** to operate at a controlled frequency to facilitate precise sealant flow control,

The sealant valve controller **908** allows control of the rate and/or volume of sealant flow. The sealant pen **902** can be constructed from polypropylene, stainless steel, Teflon™, Delrin™, or other suitable material. The sealant can create a permanent seal or a non-permanent seal, depending on the particular sealant applied. In a particular example, a sealant material such as particular polymers can be used to form a seal above a specified temperature and break the seal below specified temperatures, or vice versa.

The controller **608** can also control the sealing assembly **650** to seal a MicroChamber **602** while reducing or eliminating trapped air bubbles. For example, the sealant can be positioned in a configuration that enables bubbles to flow from the MicroChamber such as by leaving an opening in the sealant to allow trapped bubbles to escape.

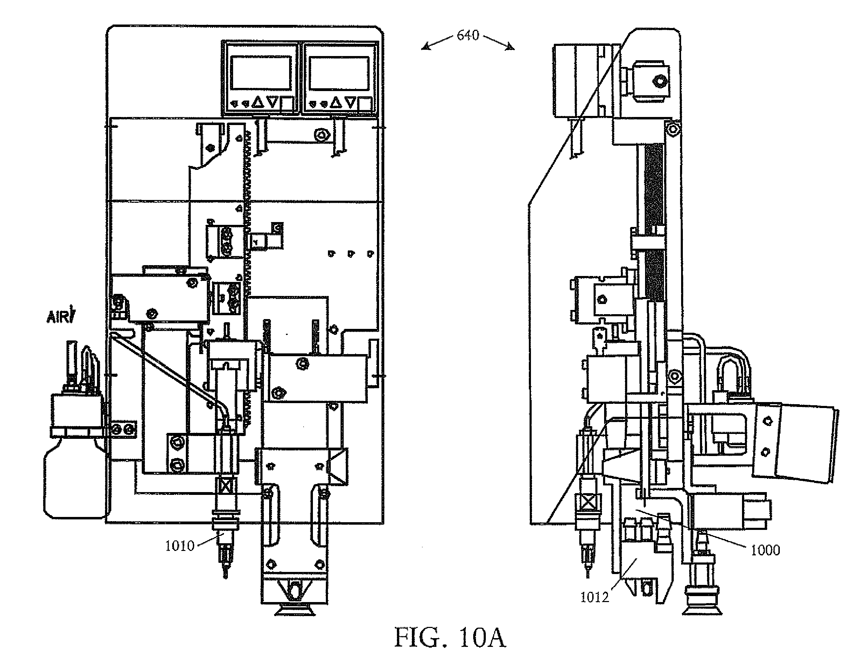

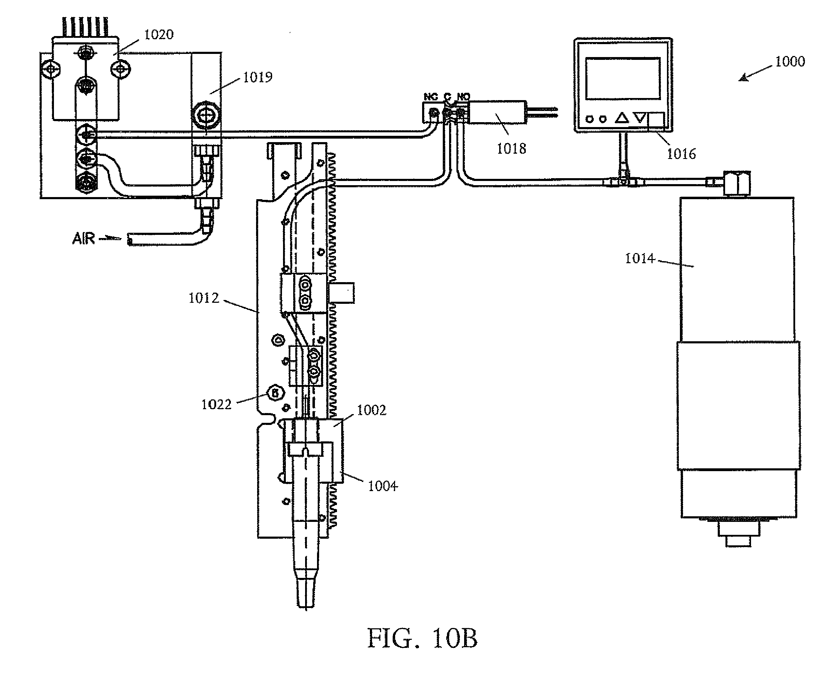

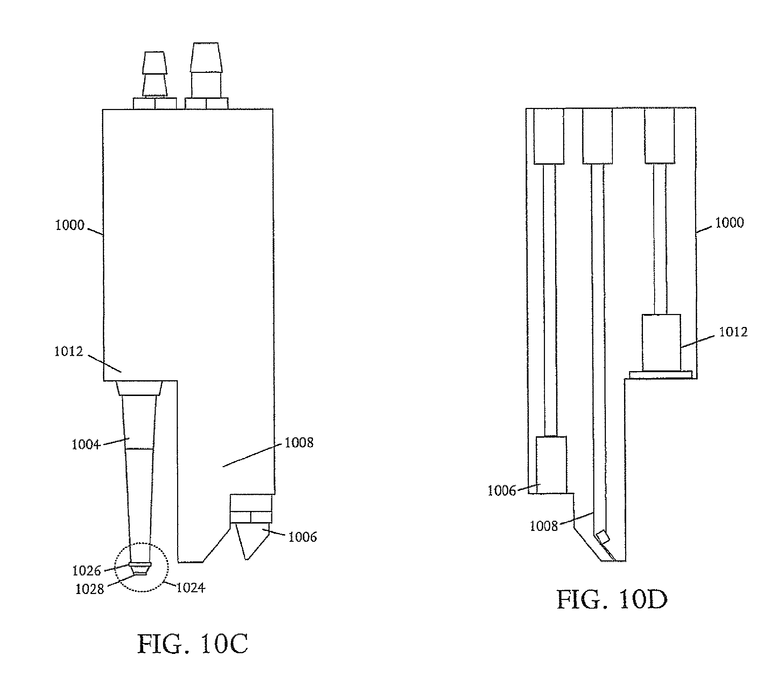

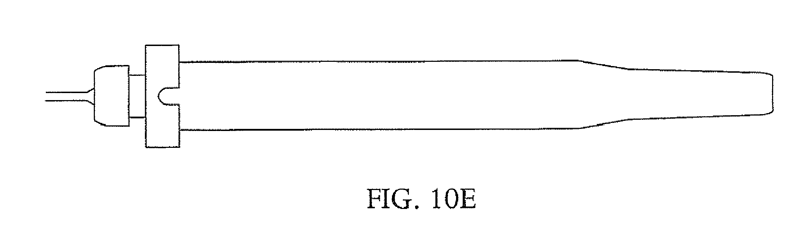

The sample processing system can aspirate a micro-volume of a selected probe and dispense the micro-volume on a sample enclosed by a barrier, such as a hydrophobic barrier, on the substrate, or on a sample on a non-barrier substrate. Referring to **FIGUREs 10A- 10E,** schematic pictorial diagrams illustrate an embodiment of a reagent dispensing device **1000** adapted for usage in the illustrative sample processing systems. **FIGURE 10A** shows an embodiment of the dispenser **1000** attached to the robotic device **640. FIGURE 10B** is a more detailed view showing the dispenser **1000** alone. **FIGURE 10C** is a side view of the reagent tip head in attachment with the robotic device **640. FIGURE 10D** shows a cross-sectional view of the reagent tip head. **FIGURE 10E** is a cross-sectional view showing a pipette tip adapter. The reagent dispensing device 1000 comprises a liquid dispenser **1002** that dispenses liquid to a chemical and/or biological sample on a substrate in a selected volume range including a capability to consistently dispense a selected liquid in volumes as low as 0.1 ul. The reagent dispensing device **1000** further comprises a pipette tip handling device **1004** coupled to the liquid dispenser **1002** that aspirates and dispenses a micro-volume of the selected liquid via a pipette tip selected from among multiple different sized pipette tips.

In a particular embodiment, the pipette tip handling device **1004** is configured to handle a plurality of pipette tip sizes including a first size with a size range from hundreds to thousands of microliters and a second size with a size range of tenths to hundreds of microliters.

The reagent dispensing device **1000** can further comprise a wash head **1006** and a blow head **1008.** The wash head **1006** is coupled to the pipette tip handling device **1004** and configured to deliver multiple selected bulk solutions in various controlled volumes to a sample substrate. In a particular embodiment, the wash head **1006** delivers a bulk solution in a range from tens to hundreds of microliters (μl). The blow head **1008** is coupled to the pipette tip handling device **1004** and configured to programmably blow air or any gas over the sample, for example to remove excess liquid from the sample.

The illustrative pipette tip handling device **1024,** attached to a robotic device **640** in the form of a Z-head that executes motion in a Z-direction, can be use two different sizes of pipette tips interchangeably by virtue of usage of a tip adapter having a two-taper design. The design enables the tip adapter **1026** to pick up more than one pipette tip size, enabling precision dispensing in a range from 0.1ul to 1000ul or more. The tip adapter **1026** can have multiple tapers to fit different size tip barrels. A photoelectric tip sensor **1028** is used to determine contact with a tip.

An illustrative wash head **1006** is capable of delivering six different bulk solutions in any volume selected by a process. The blow head **1008** dries a substrate in preparation for the next step in a process. The aspiration system can consistently aspirate and dispense any volume in a range from 0.5 mioro-liters (µl) to one milliliter (ml). In some embodiments, pipette tips can be detected using a laser sensor.

A sealant pen **1010** is configured to programmably dispense a selected amount of sealant in a programmed pattern on the sample substrate. The sealant is applied to seal a micro-chamber cover.

A reagent head **1012** is coupled to the pipette tip handling device **1004** and adapted to deliver a reagent volume to the sample substrate in a range from sub-microliters (µl) to milliliters (ml).

In some embodiments, the reagent dispensing device **1000** can be attached to a robotic head, such as a Proportional Integral Differential (PID), Proporational-Integrative (PI), or other scheme motion controller head, that can move relative to the sample substrate. The sealant pen **1010** can be coupled to the pipette tip handling device **1004** and adapted to programmably dispense a selected amount of sealant in a programmed pattern on the sample substrate. The sealant can be applied to seal a micro-chamber cover.

For example, the reagent dispensing device **1000** can be used in a sample processing system that includes a stationary platform configured to hold a plurality of substrates. The moveable robotic head can be coupled to the liquid dispenser **1002** and the pipette tip handling device **1004.** The robotic head moves relative to the substrates and is programmable to automatically process the substrates and uniformly aspirate and dispense a selected liquid micro-volume. In another embodiment, the substrate holding platform may be moving platform and the moving robotic head moves relative to the moving substrates.

In a typical application, a controller in the sample processing system can be programmed or controlled to control the liquid dispenser **1002** and the pipette tip handling device **1004.** The controller controls aspiration of a micro-volume of a fluid or reagent probe and dispensing of the micro-volume in a region constrained by a barrier containing a sample on a substrate. Similarly, the controller can control aspiration of a micro-volume of a probe and dispensing of the micro-volume on a sample on a non-barrier substrate.

Referring to **FIGUREs 6A - 6C,** the temperature control assembly **610** with active heating and cooling components can also be included in some embodiments of the sample processing system **600.** The controller **608,** or other suitable device, can be coupled to operate the temperature control assembly **610** to control the temperature environment in MicroChambers **602** by selective heating and cooling during specified phases of a protocol. The temperature of the MicroChambers **602** can be controlled individually or collectively, as specified by the process or protocol selected for the particular MicroChamber **602** or group of MicroChambers **602.**

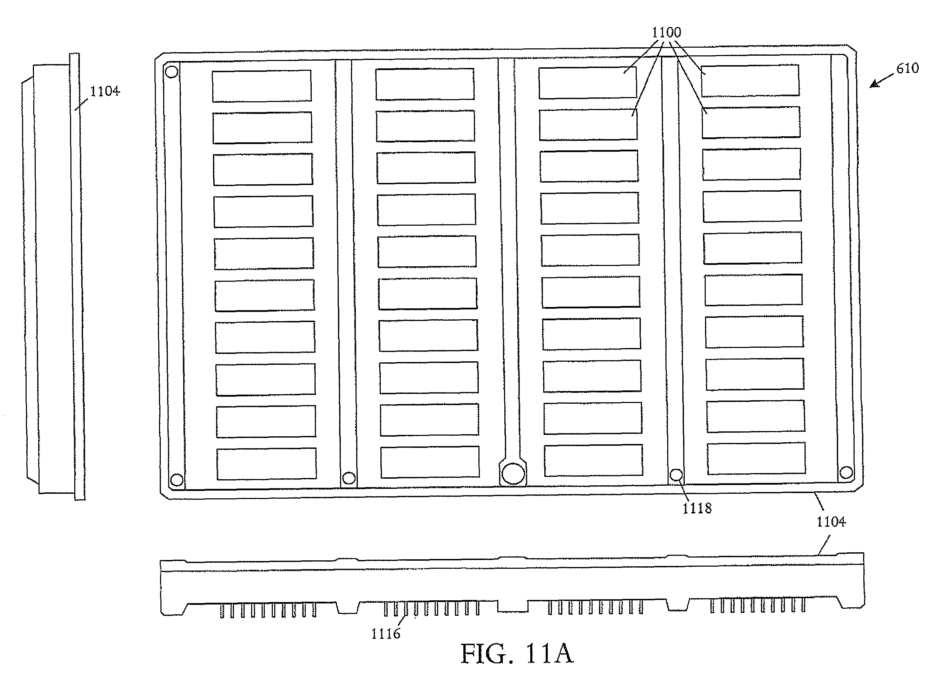

The temperature control assembly **610** can include active heating and cooling components for multiple slides or any relatively flat substrate, for example flat thermally-conductive substrates, such as glass slides, array silicon or glass chips, polymer/plastic substrates, and the like. Individual heating and cooling positions can be independently controlled. **FIGUREs 11A-11G** depict various embodiments of temperature control assemblies **610** comprising multiple temperature control elements **1100.** The platform **630** has capacity to hold multiple substrates in contact with multiple temperature control elements **1100.** The platform **630** can be removed from the sample processing system and can be used for slide storage. The platform **630** can be constructed from materials that reduce or minimize thermal convection and hold a substrate secure during removal of a MicroChamber **602** from a substrate.

The temperature control assembly **610** can enable rapid temperature response and high controllability of the individual heating and cooling positions. Rapid temperature control enables processing phases with very short duration temperature steps. The illustrative combination of heating and cooling elements, base, and heat exchangers enables a high range of dynamic temperature control, for example form -4°C to +110°C and negligible cross- talk between adjacent positions. The illustrative temperature control assembly **610** also enables performance of multiple diverse applications simultaneously.

The temperature control assembly **610** can have a relatively thin base plate on which heating elements are mounted. The base plate functions as an efficient heat sink with fins attached on a planar surface. A particular temperature control implementation may include individual slide temperature control and cycling by active active heating and cooling with solid state sensor feedback, rapid heating and cooling cycles both less than two minutes, an operating temperature of ambient to 110°C, cross-talk between elements of less than 2°C, and uniformity across the heater top from edge to center of 18°C. For example, an illustrative system that uses thermal electric heater/coolers (TEC) with active cooling can attain a temperature range of -4°C to 110°C. In other embodiments, any suitable temperature specifications may be implemented.

**FIGURE 11A** depicts top and cross-sectional views of the temperature control base assembly **1104.** The temperature control base **1104** has one or more individually controlled temperature modules. Heater assemblies are configured to heat or cool substrates such as glass slides or a flat substrate, such as a thermally-conductive substrate. In the illustrative embodiment, the temperature control base **1104** has integrated heating fins that function as heat exchangers.

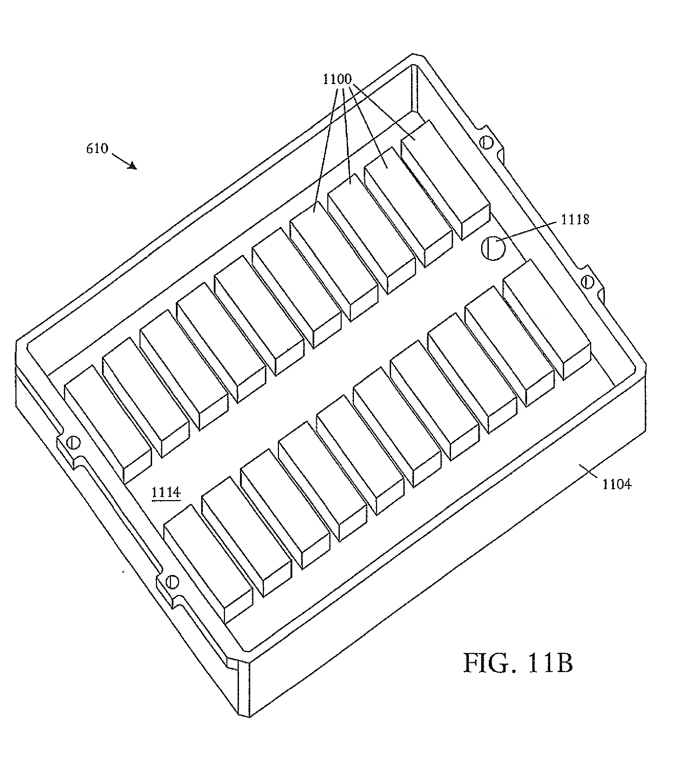

**FIGURE 11B** illustrates a perspective view of the temperature control base **1104** and an included array of temperature control elements **1100.** The temperature control base **1104** may be constructed from aluminum or other suitable material and fabricated with cooling fins that can be integrated into a fabricated part. The temperature control base **1104** can also enable fluid containment and drainage. Multiple heater assemblies can be assembled to the temperature control base **1104.**

The temperature control assembly **610** can include multiple temperature control elements **1100.** The individual temperature control elements **1100** comprise a thermally-conductive temperature application top **1102** configured to make contact to a corresponding substrate. Examples of suitable temperature control elements **1100** include resistance heaters and heat/cool Thermo-Electric Coolers (TEC).

The temperature control assembly **610** further comprises a temperature control base **1104** coupled to a plurality of individual temperature control elements **1100.** The temperature control base **1104** is typically constructed from temperature and chemical resistant polymers or metals such as polypropylene, Kynar™, Teflon™, fluoropolymers, aluminum, and stainless steel. The temperature control base **1104** can also function as a waste drain tray for process fluids.

A thermal-conducting metal plate **1106,** a temperature-sensing device **1108,** a resistive heater or thermal electric heater **1110,** and a sealed housing **1112** that thermally, chemically, and electrically isolates the individual substrate temperature control elements **1100** can be included in the temperature control assembly **610.**

The temperature control assembly **610** may further comprise a waste drain tray **1114** attached to the temperature control base **1104.** Waste liquid can be handled by gravity flow or a diverter valve and pump system to isolate hazardous and non-hazardous waste flows.

Referring again to **FIGUREs 6A-6C,** the controller **608** can control the temperature applied to individual substrate positions of the platform **630** according to sensor feedback from the temperature control assembly **610.** Various temperature control operations that can be executed by the controller **608** include, for example, executing temperature-controlled hybridization and staining simultaneously on different substrates, controlling automatic processing of DNA and protein microchips, controlling automatic processing of tissue arrays, Fluorescence In Situ Hybridization (FISH), In Situ Hybridization (ISH), and Immunohistochemistry (IHC) samples, and automatically controlling user-determined substrate temperature and incubation times. The controller can perform a combination of the various processes on a sample. Other possible operations include automatically controlling over-temperature protection and safety control, controlling active heating and cooling of the individual substrates to a selected temperature set-point, and controlling automatic active heating and cooling of a MicroChamber to a high temperature and holding the temperature for a selected time without loss of a significant quantity of fluid.

The multiple individually controllable temperature control elements **1100** can be coupled to the temperature control base **1104,** for example by integrally forming the temperature control modules **1100** into the temperature control base **1104** or installing the temperature control modules **1100** using a coupling, for example screws, bolts, clips, clasps, or other attachment or mounting devices.

The illustrative temperature control modules **1100,** for example also called temperature control elements or temperature application elements, are generally constructed from a thermally-conductive material and may be adapted for removable positioning adjacent the temperature control base **1104.**

In some embodiments, the temperature application elements **1100** can be configured for pluggable or insertable entry to a socket or connectors located on the temperature control base **1104.** In such embodiments, the temperature application element **1100** further includes a plug or connectors that connect to corresponding connectors or a socket on the temperature control base **1104.**

Optionally, in some embodiments the individual temperature control modules **1100,** for a single position in the temperature control base **1104,** may further comprise one or more vibrators **1126** that induce mixing in the MicroChambers on the substrates. In various embodiments, the vibrators **1126** may be embedded into the temperature control elements **1100,** attached to the temperature control elements **1100,** or positioned remote from the temperature control elements **1100.** The vibrators maybe mechanical oscillators, electric oscillators, piezo-electric elements, ultrasonic pulse devices, devices that produce oscillation based on application of heat, and/or other suitable devices.

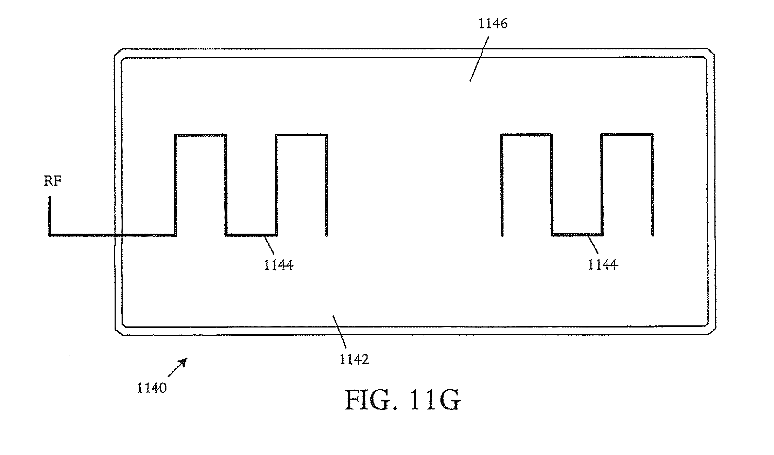

Referring to **FIGURE 11G,** a schematic pictorial diagram illustrates a view of another temperature control element embodiment **1140** that includes piezo-electric mixer or vibrator functionality. A temperature application top **1142** may also include an interdigital transducer (IDT) **1144** constructed from a material imprinted, embossed, etched, or implanted on the piezeo-electric substrate **1146.** In some embodiments, the individual temperature control module **1140** for a single position in the temperature control base **1104** may further enable the mixing functionality. The piezo-electric mixer **1148** functions according to surface acoustic wave (SAW) technology so that a radio-frequency (RF) voltage applied to the IDT **1144** creates surface acoustic waves, generating a resonant frequency that can be used to mix contents of a MicroChamber positioned on the temperature control position.

The temperature control base **1104** can be configured as a tray **1114** with peripheral sides forming a fluid containment vessel with drainage aperture **1118.** The temperature control base **1104** can be constructed from any suitable material including, for example, temperature and chemical resistant polymers or metals selected from among polypropylene, Kynar™, Teflon™, fluoropolymers, aluminum, and stainless steel. The heat exchanger **1116** is coupled, for example by attachment or integrated, into the base.

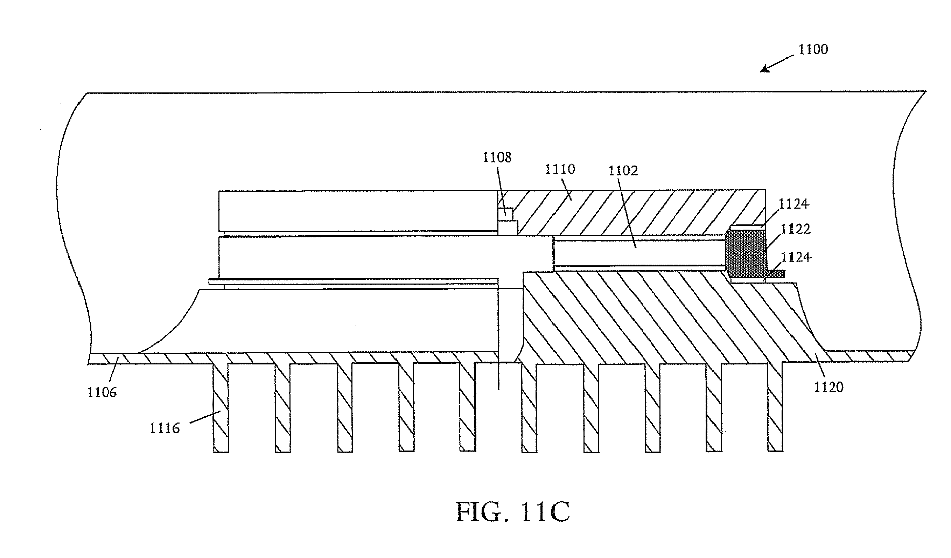

The temperature control base **1104** holds multiple temperature control modules **1100. FIGURE 11C** illustrates a cross-sectional view of a single heating and cooling module **1100.** In an illustrative embodiment, the individual temperature control modules **1100** for a single position in the temperature control base **1104** further comprise a base 1120 constructed from a temperature and chemical resistant material, a heat exchanger **1116** integrated into the base plate and constructed from the material of the base **1120,** a mount **1122** constructed from a thermally-insulating and chemical-resistant material and coupled to the base **1120,** and one or more sealing gaskets **1124** coupled to the mount **1122** and constructed from a temperature and chemical resistant material. The base **1120** is the structural material forming the temperature control base **1104.**

The illustrative temperature control modules **1100** further comprise temperature application temperature control elements **1100** constructed from a thermally conductive material and coupled to the temperature control base **1104,** a heating/cooling device **1110** secured to the temperature application top **1102;** and one or more temperature sensors **1108,** for example formed into the temperature application top **1102.**

In various embodiments, the heat exchanger **1116** may be in the form and function of cooling fins, liquid coolers, heat sinks, heat exchange coils, cooling loops, heat dissipaters, and others.

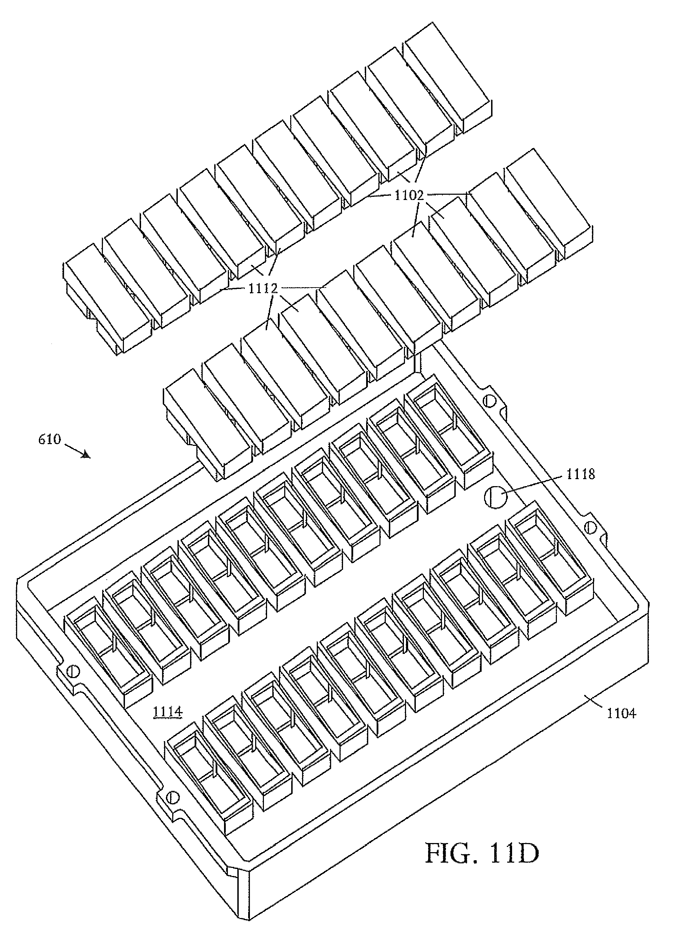

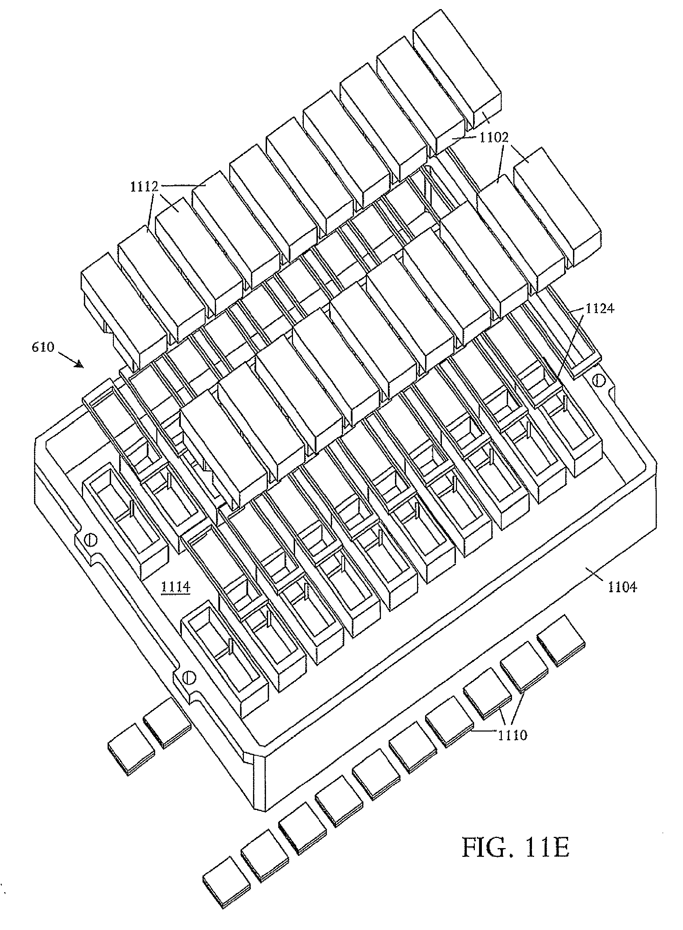

**FIGURE 11D** illustrates a perspective view of the temperature control base **1104** and temperature control elements **1100** with temperature application tops **1102** shown elevated from the temperature control assembly **610,** exposing for view the sealing gaskets **1124** and heating/cooling devices **1110. FIGURE 11E** shows a similar perspective view with the temperature application tops **1102,** sealing gaskets **1124,** and heating/cooling devices **1110** shown at different elevations, exposing the structural form of the temperature control base **1104.**

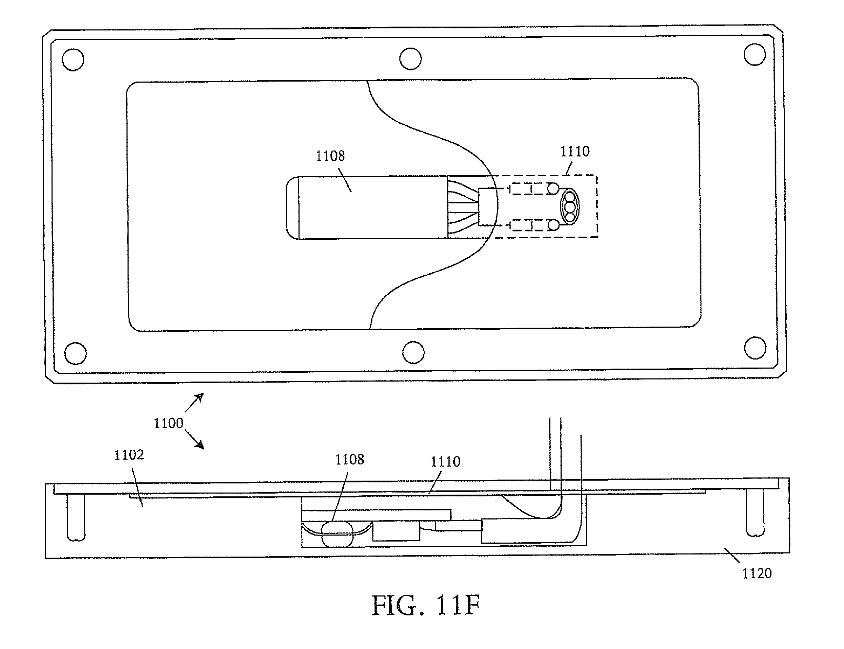

**FIGURE 11F** shows overhead and cross-sectional views of a temperature control element embodiment **1100** that uses a Kapton heating element **1110.** A sensor **1108** encapsulated in a sensor cavity with thermally-conductive epoxy. Other embodiments may use elements supporting active cooling.

In a more specific illustrative embodiment, the temperature control assembly **610** may comprise a temperature control base **1104** with multiple temperature control modules **1100.** The individual temperature control modules **1100** for a single position in the base **1120** can include a mount **1122,** one or more sealing gaskets **1124,** a temperature application top **1102,** a heating/cooling device **1110,** and one or more temperature sensor **1108.** The mount **1122** may be constructed from a thermally-insulating and chemical-resistant material such as ceramic, Viton™, Kynar™, PEEK™, and Teflon™. One mount **1122** is included for a heating/cooling element position. The sealing gaskets **1124** are coupled about the mount **1122** and constructed from fluorocarbon rubber, Viton™, or other heat and chemical resistant material. One or more gaskets **1124** may be included for each position. The temperature application top **1102** may overlie the temperature control base **1104** and be constructed from a thermally conductive material such as ceramic, aluminum, brass, copper, or alloy of aluminum, brass, or copper. One temperature application top **1102** is included for a heating/cooling element position. The heating/cooling device **1110** may be a Peltier heating/cooling device, a Thermo-Electric Cooler (TEC), or resistive heater secured to the temperature application top **1102.** The temperature sensors **1108** may be integrated circuit (IC) sensors, thermocouples, or thermistor-type temperature sensors sealed into a cavity in the temperature application top **1102.** Typically, each position has a single heating/cooling device **1110.**

In an illustrative embodiment, heater assemblies contain a thermally-conducting metal plate such as constructed from aluminum, copper, or brass, a temperature sensing device, and a resistive or thermal electric heater. The components are sealed, potted, or molded with a temperature and chemical resistant compound that is also a thermal conductor and electrical insulator. The embodiment may further include a slide carrier and grated barrier slides. The slide carrier can be constructed of an aluminum frame or thermal insulating plastic inserts. The insert reduces thermal cross-talk between slides. The slide carrier and barrier slides can be positioned in the system working space.

Referring to **FIGUREs 6A-6C** In conjunction with **FIGUREs 11A-11G,** the controller **608** is communicatively or controllably coupled to the temperature control assembly **610** and independently sets temperature and controls temperature cycling for individual temperature control elements **1100** and associated substrates and samples of the multiple substrate positions.

The controller **608** manages the sample processing system **600** and the temperature control assembly **610** in combination to independently perform multiple, diverse applications simultaneously for individual substrates of the multiple substrates with negligible cross-talk between adjacent substrates. The structure enables independent programming of heating and/or active cooling in individual temperature control modules.

If a large number of slides are to be processed, a run need not be restarted between processing of various batches. Slide racks holding finished slides can be removed and racks holding fresh slides can be introduced during an existing run. Otherwise, slides having higher priority of handling can be introduced into the system without aborting an existing run and can be completed before the slides already being processed.

In accordance with another embodiment of the illustrative system, the controller **608** controls the temperature control assembly **610** for independent control and monitoring of multiple individual channels, corresponding to the multiple temperature control elements **1100.** A heating and cooling device **1100** shown in **FIGUREs 11A-11G** is configured to receive a substrate holding a chemical and/or biological sample and the controller **608** controls current magnitude and direction in the heating and cooling devices **1110** using proportional-integrative control based on a first term proportional to error between averaged temperature and a set point and a second term of the error summed over time.

The controller **608** executes a control process that reads a temperature measurement from the heating and cooling device **1100** for the individual channels, samples the temperature measurement with a time constant in a range of hundreds of milliseconds, computes an error term as the difference between the set point and the temperature measurement, and computes the first term and the second term using the error term.

In a particular implementation, temperature is read 200 times per second and stored in a 40-word circular buffer, resulting in a time constant of about 200 milliseconds. Ten times per second, the control process is run and averages the most recent forty temperature readings to obtain temperature T. In other implementations, any suitable sampling frequency and time constant may be used.

The controller 608 can also execute a control process that computes an output response according to equation (1) as follows:

$$Output = (G * err) + (G * Ki * \textstyle\sum err), \qquad (1)$$

where *err* is equal to set point minus the temperature measurement, Σ*err* is continuous running sum of the error, *Ki* is a multiplicative parameter, and *G* is an overall gain parameter. The output response can be limited to values between a specified positive heating limit and a specified negative cooling limit. The integral Σ*err* can be set to zero if the first term *(G * err)* exceeds the largest magnitude of the positive heating limit and the negative heating limit. In some embodiments, overshoot may be reduced by terminating summing of Σ*err* if either *err* is greater than a selected windup threshold, or the second term *(G *Ki *Σerr)* exceeds the largest magnitude of the positive heating limit and the negative heating limit.

In typical operation, at equilibrium, for example with only small error, the integral term predominates. When the set point changes, the proportional term rapidly becomes very large. The temperature approaches the set point only after a response time. During the response time, the integral, if allowed to increase, can become even larger and cause the temperature to drive far past the set point and leading to a large overshoot. To prevent an unstable condition, the integral is zeroed and summing of the integral is terminated while the error is large.

A controller operates the temperature control assembly **610** by issuing several commands including global commands and channel-specific commands. Global commands include a read current command and a power on/off command. Channel-specific commands include: (1) set temperature set point, (2) read temperature, (3) enable / disable output, (4) read all channels, (5) set proportional-integrative (PI) parameters, and read PI parameters.

The read current command returns a measured current consumption in amperes of a heating/cooling device controller. The power on/off command turns on and off a power relay, depending on current status. The power relay controls voltage to the power output section of the heating/cooling device **1110.** All functions including temperature readout are operational even when the power relay is in the off setting since only output power is disabled.

The set temperature set point command sets a target temperature for a channel. If the power relay is on, then channel output is enabled and the controller attempts to attain and maintain the set point temperature. Read temperature returns a single channel temperature readout. Enable / disable output performs the operation of enabling or disabling the channel output. In the disabled state, the temperature readout remains active and the set point may be changed, but no current flows through the heating/cooling device **1110.** In the disabled state, the temperature does not regulate. The read all channels command reads all channels at one time, returning a large packet containing either temperature or set point data along with channel status flags for each channel. Read PI parameters reads back any requested PI control algorithm parameter. Set PI parameters sets the PI control algorithm parameter to the specified value. The parameters include overall proportional term gain (G), an integrative term multiplicative factor (Ki), windup threshold (W), positive threshold (M+), negative threshold (M-), and set point (SP).

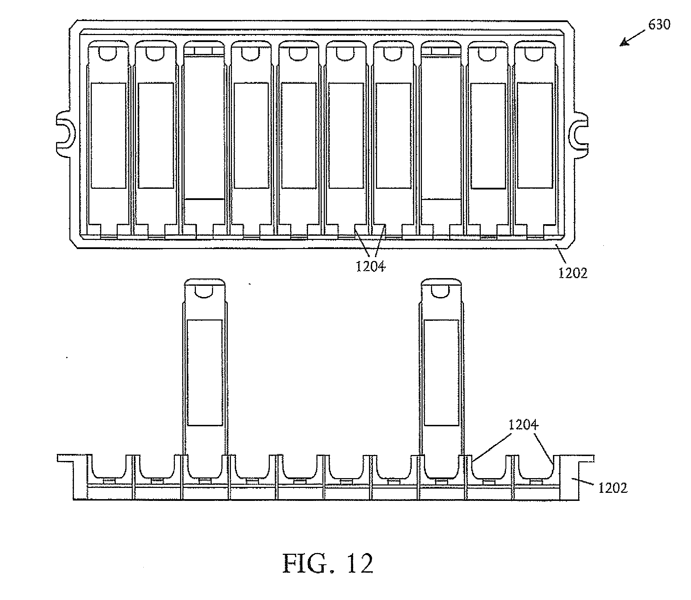

Referring to **FIGURE 12,** a pictorial diagram illustrates an embodiment of the platform **630,** comprising a carrier frame **1202** and a plurality of inserts **1204** that reduce thermal cross-talk between the substrates. The carrier frame **1202** is constructed from a material, such as aluminum, and the inserts **1204** are constructed from aluminum or thermal-insulating plastic.

The substrate or slide carrier enables multiple substrates, for example ten slides, to be placed together onto the substrate positions while holding the substrates in contact with temperature application tops, and prevent the substrates from being pulled out of the carrier during removal of the MicroChamber covers. The platform is generally constructed of materials that reduce or minimize thermal convection for more efficient temperature control.

Referring again to **FIGUREs 6A-6C,** some embodiments of the sample processing system **600** can also include one or more mixers **612** adapted to mix the contents of the MicroChambers **602** collectively, and/or individually. The controller **608,** or other suitable mixing control device, can be coupled to control operation of the mixer(s) **612**. An environment mixing system **658** may be implemented in the sample processing system **600**. The environment mixing system **658** comprises the sample processing system **600** configured to mix an environment within a MicroChamber **602**.

In some embodiments, a vibration motor **660** can be included that is positionable in the vicinity of the Micro-Chamber **602**. The controller **608** is communicatively coupled to the vibration motor **660** and adapted to generate a vibration in the MicroChamber **602**.

In other embodiments, the robotic device **640** is adapted to move relative to the MicroChamber **602** and a member, for example one or more cushioned members, needles, pens, pipettes, or other items attached to the pipette tip handling device **652** is attached to the robotic device **640**. The controller **608** controls the robotic device **640** to a position to generate a vibration in the MicroChamber **602**. For example, the MicroChamber cover(s) can simply be touched once or repeatedly to send a pressure pulse through the microenvironment.

In further embodiments, a piezo-electric transducer **662** can be included that is positionable in the vicinity of the MicroChamber **602**. The controller **608** is communicatively connected to the piezo-electric transducer **662** and adapted to generate a vibration in the MicroChamber **602**.

In other embodiments, the temperature control assembly **610** has active heating and cooling capability and is positionable in the substrate vicinity. The controller **608** controls the temperature control assembly **610** and programmed to generate a motion in the microenvironment by temperature cycling.

Other suitable vibration techniques and devices can be utilized to mix the contents of the MicroChamber(s) **602**.

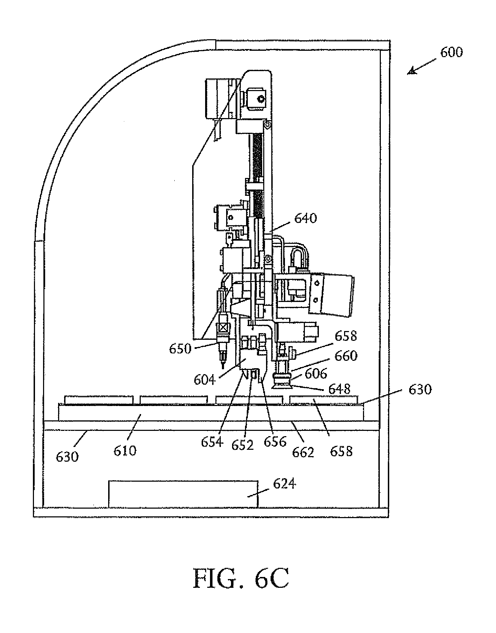

The sample processing system **600** in **FIGUREs 6A** and **6B** is shown without an enclosure to enable viewing of a robotic device **640** and other internal components, devices, and parts. **FIGURE 6C** shows an enclosure **664** around components on the platform **630** of the sample processing system **600**. The enclosure **664** can be fabricated with transparent material to allow an operator to view operation of the processing system **600**. The enclosure **664** can also help protect the MicroChambers **602** from contamination, as well as help control the microenvironment of the Micro-Chambers **602**.

A tip disposal orifice **622** can be included in some embodiments of the sample processing system **600** to allow the robotic head **640** to discard used pipette tips 626. A tip disposal bin **624** can be located adjacent the tip disposal orifice **622** to store discarded pipette tips **626**. A horizontal bar **628** can be located at the center of the tip disposal orifice **622** to help prevent discarded pipette tips **626** from stacking and blocking the disposal orifice **622**. A drain bin **632** can be positioned under the platform **630** for draining fluids to a waste container.

A pipette tip holder can also be included in some embodiments of the sample processing system **600** and configured with one or more pipette tip racks **634A** and **634B** capable of containing arrays of pipette tips **626**. A reagent vial holder **636** is shown adjacent the pipette tip racks **634A** and **634B** and can be affixed to the platform **630** or adapted to be removable from the platform **630**.

A slide holder **629** can also be included in some embodiments of the sample processing system **600** to hold one or more substrates or slides for future use. Another slide holder can be included to store used substrates or slides.

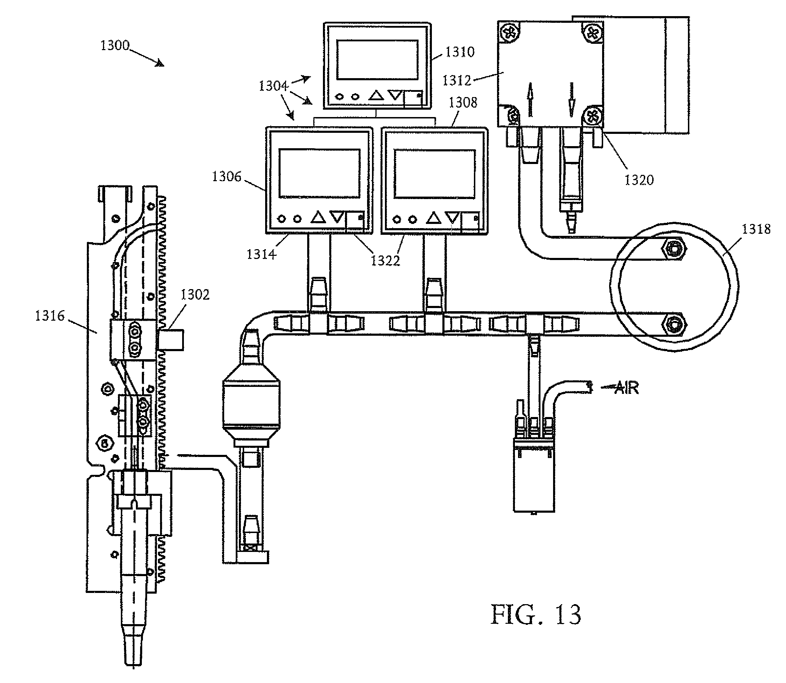

Referring to **FIGURE 13,** a schematic pictorial diagram illustrates an embodiment of a fluid handler **1300** for usage in a sample processing system. The fluid handler **1300** comprises a fluid dispenser **1302** and a fluid level detector **1304**. The fluid dispenser **1302** is operative in a system for handling chemical and/or biological samples. The fluid dispenser **1302** is adapted to dispense one or more selected fluids to a selected sample. The fluid level detector **1304** comprises a combined vacuum detector 1306 and pressure detector **1308**. The liquid level of a reagent can be sensed using the vacuum system to avoid creation of bubbles in the reagent, thereby introducing sensing uncertainty in the fluid level.

The fluid dispenser **1302** can further comprise a controller **1310** that communicates with the fluid level detector **1304** and is adapted to selectively operate the vacuum detector **1306** to measure fluid level for relatively large volume, low viscosity fluids. Similarly, the controller **1304** selectively operates the pressure detector 1308 to measure fluid level for relatively low volume and high viscosity fluids.

The fluid handler **1300** further comprises a metering pump **1312** that is cycled to create a vacuum source, a vacuum switch **1314** for detecting a pressure change, and the controller **1310** that is adapted to receive a signal indicative of the change in pressure. The fluid handler **1300** can further comprise a robotic handler **1316** adapted to manipulate a pipette including a pipette tip. The controller **1310** executes a control operation that includes lowering the pipette into a fluid container, receiving a signal from the vacuum switch **1314** upon detection of the pressure change when the pipette tip touches the fluid surface in the container, saving pipette position information at the pressure change, and determining the distance to move the pipette to aspirate a selected fluid volume.

The pressure detector **1308** also uses the metering pump **1312** and controller **1310** along with a pressure switch **1318** that determines a positive pressure on detection of a pressure change. The controller **1310** receives a signal indicative of the pressure to determine fluid level. The controller **1310** executes a control operation that includes lowering the pipette into a fluid container, receiving a signal from the pressure switch **1318** indicative of positive pressure to determine when the pipette tip touches or nearly touches the fluid surface in the container, saving pipette position information at the pressure change, and determining the distance to move the pipette to aspirate a selected fluid volume.

The fluid handler **1300** can also include the sample processing system which is adapted to dispense at least one reagent fluid to a chemical and/or biological sample. One or more reagent/probe containers contain fluids to be dispensed during sample handling. The fluid dispenser **1302** and fluid level detector **1304** can be used to determine fluid level in the reagent/probe containers.

The controller **1310** can mange the fluid level detector **1304** to select between operation of the vacuum detector **1306** and the pressure detector **1308** to reduce or eliminate bubbles in the liquid. For example, usage of the vacuum detector **1306** reduces formation of bubbles for relatively large volume, low viscosity fluids.

According to another embodiment of the fluid handler **1300,** the sample processing system is adapted to apply at least one selected reagent to a chemical and/or biological sample in an automated process. The sample processing system includes one or more reagent/probe containers. A vacuum and pressure source **1320** is coupled to the sample processing system and used to dispense fluids. A vacuum and pressure sensor **1322** is coupled to the sample processing system and used to measure a condition of the reagent/probe containers. The fluid level detector **1304** operates in conjunction with the vacuum and pressure source **1320,** vacuum and pressure sensor **1322,** and fluid level detector **1304** and detects fluid level in the reagent/probe containers selectively based on either vacuum or pressure changes.

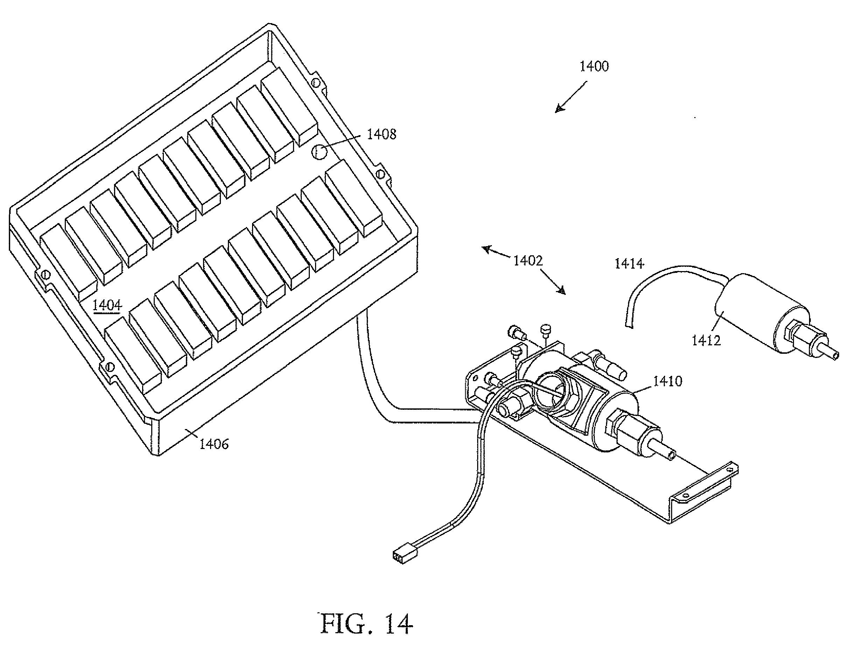

A toxic reagent in a process can become a waste product. Toxic waste can be separated from non-toxic waste to reduce the volume of toxic waste for disposal. Referring to **FIGURE 14,** a schematic pictorial diagram illustrates an embodiment of a waste handling system **1400** that can be used in a sample processing system. The waste handling system **1400** comprises a sample processing system configured to apply at least one selected reagent to a chemical and/or biological sample on a substrate in an automated process and a waste separation system **1402.** The waste separation system **1402** is adapted to divert effluent waste from the sample processing system into a plurality of separate parts under program control.

The waste separation system **1402** comprises a platform **630,** a waste drain tray **1406** coupled to the platform **630** and having an outlet **1408,** a multiple-way diverter valve **1410** coupled to the waste drain tray outlet **1408,** a pump **1412,** and drain lines **1414.** The pump **1412** programmably separates the effluent.

A controller can be coupled to the sample processing system, the multiple-way valve **1410,** and the pump **1412** and can be adapted to programmably automate application of reagent dispensing and separation of effluent in a combined operation. Based on chemicals specified by a protocol, the controller switches the diverter valve **1410** directing waste to hazardous or non-hazardous waste receptacles. The pump **1412** assists waste flow to the receptacles. The controller can perform automatic separation of toxic from non-toxic waste in combination with control over other operations on the sample, such as dispensing of reagents and washing. The controller redirects fluid by switching the valve **1410,** for example using software commands. For example, the controller can control dispensing of the reagents based on information relating to toxicity of the particular reagents. For known toxic reagents, the controller can control the multiple-way valve **1410** to direct the fluid to a toxic disposal container. Similarly, for non-toxic reagents, the controller manages the multiple-way valve to direct the effluent to a non-toxic disposal container. In another application, the controller can automate application of multiple reagents to samples and separation of multiple waste effluents into different effluent waste receptacles in a combined operation.

In some embodiments, the waste handling system **1400** can include a platform **630** that further includes, referring to **FIGUREs 11A-11G** in combination with **FIGURE 14,** a temperature control temperature control base **1104** coupled to a plurality of individual substrate temperature control assemblies **1100,** and a waste drain tray **1114** coupled to the temperature control temperature control base **1104.** The waste drain tray **1114** has an outlet or drain **1118.** The multiple-way valve **1410** is connected to the waste drain tray outlet **1118** and can be programmed to controllably separate the effluent by gravity flow.

The components in the sample processing system **600** can be arranged in specified locations and orientations relative to the robotic arm **640.** The configuration of the components can be specified by a user via a user interface, or may be preprogrammed. Knowledge of the location and orientation of the components allows more accurate and efficient operation of the robotic head **640.** Additionally, configuration of the sample processing system **600** can be adapted for particular processes or protocols to be performed. For example, the operator can customize the configuration to accommodate specified sizes and numbers of covers, pipettes, MicroChambers **602,** and reagents, among others.

Additionally, in some embodiments, the components in the sample processing system **600** can include features such as an encoder or RFID tag that allows the identity of the components, the position of the components on platform **630,** and other characterizing information about components to be determined without requiring the information to be preprogrammed or input by the operator. The controller **608** can use the information to accurately position and operate the robotic device **640** relative to the other components in the sample processing system **600.**

Further, components in the sample processing system **600** can include bar codes, an RFID tag, or other identifier than can be detected by sensors and/or signal receivers in or near the sample processing system **600.** The controller **608** or other suitable computer processing device can include logic that allows the location of the components to be tracked and recorded. For example, a reagent container **644** may be taken from one sample processing system **600** and installed in another sample processing system **600.** If the component, such as the reagent container **644,** includes an identifier, the controller **608** can identify the component and provide information regarding the new location of the component to an operator. Additionally, the system **600** from which the component was removed can detect the event and can issue a suitable alert, such as a text or voice message to the operator, when a process is selected that requires replacement of the component.

Two or more of the sample processing systems **600** can be coupled to a network to provide and receive information from each other. Information from each system **600** can be collected, and logs and statistics regarding the operation of one or more of the systems **600** can be provided from each processing system **600** and/or from any other system configured to communicate with the network of systems **600.** Any suitable communication interfaces and protocols can be utilized to form a network of the systems **600.**

The robotic device **640** can be moved to different locations over the platform **630** by the action of motors that operate in combination with sliding tracks to precisely position the robotic device **640** at a specified location. An X-axis track 638 is shown as the principal lengthwise horizontal axis of the apparatus. A single X-axis track **638** is supported at either end on bearing shafts and brackets. A Y-axis track 664 allows movement of the robotic head **640** in a second dimension. Movement of the tracks 638, **664** is enabled by motors operated by a controller, computer, or other suitable device. The Z-axis is orthogonal to the X and Y axes. Additional X-axis tracks 638 and Y-axis tracks **664,** or other suitable structure can be included in the sample processing system **600** to allow one or more additional robotic devices **640** to move independently of one another and reduce the amount of time required to process multiple chambers **602.**

Flexible electronic leads and tubing, including both gas and liquid supply conduits, can lead from the robotic device **640** to appropriate fluid reservoirs and/or electronic control equipment. The supply conduits are suitably long, flexible and durable and can originate from various pumps. The supply conduits can pass through a flexible wire carrier on one side of X-axis track **638,** and through a wire carrier at the top of the X-axis track **638** to conduct supply conduits to movable arm **614** and robotic device **640.**

In an illustrative system, power can be supplied to the sample processing system **600** by alternating current (AC) to direct current (DC) medical-grade power supplies. Most functions, other than high-power operations such as compressor and blower functionality, can operate on low voltage DC power. In a typical embodiment, an air compressor may supply a linear, regulated range of 3-6 pounds per square inch (PSI) or other suitable pressure, with a blower with linear control, open 0.7 cubic feet per minute (CFM), and a vacuum with open condition of 450-0 mm/Hg. Any other suitable compressor, blower, and vacuum specification may be implemented.

In some embodiments, the robotic device **640** may include a closed-loop or open-loop motion controller that uses a Proportional Integral Differential (PID) or Proportional-Integrative algorithm, and or other process control algorithms to perform motion control. In some embodiments, the robotic device **640** can be positioned with accuracy in the X and Y axes to 0.2 mm, and positional accuracy in the Z-axis to 0.4mm, however, the processing system **600** can be configured with components that achieve other levels of accuracy.

Motors or other suitable drive components move the movable arm **614** under computer control, enabling programming of arm movement between various work locations on the platform **630.** The robotic device **640,** which can include a hollow tip head to dispense liquids or gasses through the robotic device **640** to the MicroChambers **602.** In some embodiments, the movable arm **614** is configured with either multiple, permanently attached tips with different functions or multiple disposable tips located on the movable arm **614** concurrently. For example, a single hollow channel in the robotic device **640** may have multiple channels connected to separate pumps with individual tips having possibly different functionality attached. A portion of the robotic device **640** can be adapted to pick up pipette tips **626** from the standard tip racks **634A, 634B.** The pipette tips **626** can be oriented in the tip racks **634A, 634B** to allow the tip handling device **652** to engage the base of the tips **626** for insertion onto the robotic device **640.** The tips **626** can be arranged in an array so individual tips **626** in the racks **634A, 634B** are accessible to the user and the robotic device **640.**

In some embodiments, the pipette tip handling device **652** can use two different sizes of pipette tips interchangeably by virtue of usage of a tip adapter having a two-taper design. The design enables the tip adapter to pick up more than one pipette tip size, enabling precision dispensing in a range from 0.1nanoliters (nl) to 1000 μl or more. The tip adapter can have multiple tapers to fit different size tip barrels. A sensor or other suitable device can be used to determine contact with a pipette tip **626.** The level of substance remaining can be used to check the accuracy of countdown volume for the particular container **644.**

The movable arm **614** can be controlled to move to a particular predetermined location and carry out a preselected motion or other operation, such as grasping or releasing a tip **626** from the robotic device **640.** Standardized motions of the arm **614** can be programmed so that individual MicroChambers **602** at specific predetermined locations on the platform **630** can be treated with reagents and/or wash fluids obtained from reagent containers **644** or other substances supplied through the robotic device 640. The amount of reagent or other substances used can be tracked as inventory information that can be shared among networked processing systems **600** and accessed by operators.

During operation multiple MicroChambers **602** are placed in a tray that is inserted at a predetermined location, usually according to registration pins in the tray so that individual MicroChambers **602** are always located in the predetermined relative positions on the platform **630.** The sample processing system **600** is programmed appropriately for operation with components placed at predetermined or determinable locations.

The robotic device **640** can also be configured with a wash head 654 and a blow head **656.** The wash head **654** is capable of delivering one or more different bulk solutions in any volume selected by a process. The robotic device **640** can apply liquid to a MicroChamber **602** from a wash buffer reservoir via a liquid supply conduit to the wash head **654.** The blow head **656** can be used to remove excess buffer from the MicroChamber **602** prior to subsequent processing. Removal can be performed by blowing gas through the blow head **656** while the robotic device **640** travels along the X, Y, and/or Z axes without disrupting the contents of the MicroChamber **602.** A small amount of buffer can be left on the MicroChamber **602** to assist in reagent distribution.

In some embodiments, the wash head **654** can dispense fluid in a range from 35$\mu$l to 610$\mu$l. In other embodiments, any suitable fluid volume may be dispensed. The wash head **654** may be constructed from any suitable material such as acrylic and/or stainless steel and can be mounted on a linear slide to enable back and forth washing motion.

The robotic device **640** can be controlled to engage a pipette tip **626** from the pipette tip racks **634A, 634B,** move the pipette tip **626** to a selected reagent container **644,** and draw a selected amount of reagent using vacuum suction. The sample processing system **600** can be programmed for efficient operation to deliver the particular reagent to multiple specimens that are to be treated with the reagent. The robotic device **640** can be moved to the appropriate specimens to dispense the reagent in a pre-assigned pattern that operates in combination with a thin liquid film on the MicroChamber **602** to assure spreading of the reagent over the entire surface of the MicroChamber **602.**

The disposable pipette tip **626** can then be discarded and the movable arm **614** moves the wash and blow heads to apply buffer and then remove excess buffer from the next group of MicroChambers **602** to be processed, while the prior group of MicroChambers **602** are incubated with the reagent. The robotic device **640** can engage the next available disposable tip from the tip rack and a selected reagent can be drawn into the tip and applied. Selected steps can be repeated until all specified MicroChambers **602** are treated with reagent or reagent incubation is complete and reagents may be removed.

In some implementations, the controller **608** can be integrated with other processing components in the sample processing system **600,** or in a stand-alone computer, for example running a common operating system such as Windows NT™, 2000™, XP™, or others. In a typical implementation, the controller **608** can run multiple, different, protocols simultaneously. For example, in some embodiments staining can be implemented in a possible platform of forty slides, thirty bottle reagents, and ninety-six well-plate reagents. Any suitable configuration may be implemented. The controller **608** can enable either open runs or barcode runs. Built-in factory protocols may be implemented as well as user-defined or custom protocols.

The controller **608** can generate a user interface that allows an operator to preview an entire protocol and edit selected portions of the protocol before starting the process. A slide map may be displayed showing real-timer protocols, processing, and progress. The system can display run-time and time to completion. Details of current operation can be displayed in a status box. The system **600** can support print reports for slide workload, reagent workload, missing reagents in a barcode run, insufficient reagents in a barcode run, expired reagents in a barcode run, assay reports, run logs, and the like.

In some embodiments, the controller **608** may enable set-up of subsequent open runs only during a current run. Reagent volume countdown may be performed for a run and/or a series of runs. Audio and visual alarm features may be included for protocol run completion and system errors. The system **600** may support pause and stop function icons on various or all graphical user interface displays. Safety confirmation may be displayed when deleting slides. The system may support a virtually unlimited number of protocol steps. The system may support optimization of priming location for the robotic device **640** and keyboard functions to move the robotic device **640** during calibration. A save function may be implemented for the calibration procedure.

A single-user login may be supported. A drop-down list box may be displayed for multiple specimen types and user names. A user-defined reagent dispensing pattern may be supported on a substrate during a run.

In some embodiments, the system **600** can search for subsequent pipette tips if a tip is missing. A start/cancel delayed start function can be implemented after programming a protocol. The system **600** can enable editing of single slides in the barcode and open formats, and may enable multiple edits in the barcode and open formats. The system **600** can support a capability to buffer slides before and after runs. The system **600** may further support an error message history file and a protocol control-login requirement for particular defined protocol modifications.

Various features of the system **600** enable aspects of walk-away automation. For example, the controller **608** can use a STAT feature that allows the operator to prioritize processing of one or more selected samples.

Similarly, the controller **608** can enable a continuous access capability including a capability to remove a finished sample or a sample in the middle of a run and replace the sample with another while processing of other samples continues.

While the present disclosure describes various embodiments, these embodiments are to be understood as illustrative and do not limit the claim scope. Many variations, modifications, additions and improvements of the described embodiments are possible. For example, those having ordinary skill in the art will readily implement the steps necessary to provide the structures and methods disclosed herein, and will understand that the process parameters, materials, and dimensions are given by way of example only. The parameters, materials, and dimensions can be varied to achieve the desired structure as well as modifications, which are within the scope of the claims. For example, although particular systems are described that include many novel features, each of the different features may be implemented in a system that either includes or excludes other features while utility is maintained.

In the claims, unless otherwise indicated the article "a" is to refer to "one or more than one".

**Claims**

1. An apparatus comprising:

   a sample processing system configured to create a Micro-chamber containing a sample on a substrate.

2. The apparatus according to claim 1 further comprising:

   a reagent dispensing device configured to apply one or more reagents to the sample; and
   a cover handling device operable in combination with the reagent dispensing device to automate placement and removal of Micro-chamber covers on the substrate.

3. The apparatus according claim 1 further comprising:

   a cover dispenser that individually dispenses Micro-chamber covers, the dispenser being capable of dispensing covers of multiple different sizes;
   a robotic head adapted to move relative to the cover dispenser and the substrate; and
   an effector coupled to the robotic head that programmably grips and releases Micro-camber covers, the effector being programmable to perform multiple functions including removing a Micro-chamber cover from the cover dispenser, moving the Micro-chamber cover to a specified position, placing the Micro-chamber cover on the substrate, and removing the Micro-chamber cover from the substrate.

4. The apparatus according to claim 3 wherein:

   the effector further comprises at least one of the group consisting of: a vacuum pad, an electromagnetic attachment device, and a mechanical robotic attachment device, each of which is configured to grip and release the Micro-chamber covers.

5. The apparatus according to claim 3 further comprising:

   a controller that controls the robotic head and effector;
   a program code executable on the controller and configured to control automatic removal of a Micro-chamber cover from the cover dispenser; and
   a program code executable on the controller and configured to control automatic placement of a Micro-chamber cover in a manner that minimizes air bubble creation and encloses fluid on the substrate.

6. The apparatus according to claim 3 further comprising:

   a controller that controls the robotic head and effector; and
   a program code executable on the controller and configured to control automatic Micro-chamber cover movement and placement on a barrier laterally containing a sample to create a Micro-chamber on the substrate.

7. The apparatus according to claim 3 further comprising:

   a controller that controls the robotic head and effector; and
   a program code executable on the controller and configured to control at least one of: automatic formation of a barrier on the substrate; Micro-chamber cover movement and placement on a non-barrier substrate to create a Micro-chamber; and automatic removal of the Micro-chamber cover from the substrate.

8. The apparatus according to claim 3 further comprising:

a controller that controls the robotic head and effector;
a sealing assembly further comprising a sealant pen, a sealant reservoir coupled to the sealant pen, a sealant valve controller, and a sealant pen valve that is manipulated by the robotic head to selectively eject a pattern of sealant and seal a Micro-chamber; and
a temperature control assembly with active heating and cooling that is independently programmable for an individual substrate of a plurality of substrates.

9. The apparatus according to claim 8 further comprising:

a program code executable on the controller and configured to control automatic formation of a Micro-chamber on the substrate.

10. The apparatus according to claim 8 further comprising:

a program code executable on the controller and configured to control automatic placement of a fluid micro-volume on the substrate within the Micro-chamber.

11. The apparatus according to claim 8 further comprising:

a program code executable on the controller and configured to control automatic sealing of fluid within the Micro-chamber.

12. The apparatus according to claim 8 further comprising:

a program code executable on the controller and configured to control automatic heating of the substrate while preventing fluid evaporation from the Micro-chamber.

13. The apparatus according to claim 3 further comprising:

one or more Micro-chamber covers having an epoxy pattern printed on one side to prevent sticking of adjacent Micro-chamber covers in the cover dispenser.

14. The apparatus according to claim 1 further comprising:

the plurality of substrates printed with a Teflon™ pattern in a configuration that reduces incidence and magnitude of air bubbles trapped under a Micro-chamber cover.

15. The apparatus according to claim 1 further comprising:

a robotic head adapted to move relative to the substrates;
a pipette tip handling device coupled to the robotic head that handles a plurality of different sized pipette tips;
a wash head that delivers a plurality of selected bulk solutions in a plurality of controlled volumes to a sample substrate; and
a blow head that programmably dries the sample substrate.

16. The apparatus according to claim 15 further comprising:

a sealant pen adapted to programmably dispense a selected amount of sealant in a programmed pattern on the substrates, the sealant being applied to seal a Micro-chamber cover.

17. The apparatus according to claim 1 further comprising:

a stationary platform configured to hold a plurality of substrates; and
a moveable robotic head adapted to move relative to the substrates and is programmable to automatically process the substrates and manipulate the Micro-chamber covers.

**18.** The apparatus according to claim 1 further comprising:

a controller that controls the sample processing system and the cover handling device to automate a process of dispensing a fluid micro-volume on the substrate and covering the fluid micro-volume with a Micro-chamber cover to create a Micro-chamber on the substrate for chemical, biological, genomics, proteomics, histology, or cytology assays.

**19.** The apparatus according to claim 1 further comprising:

a temperature control assembly with active heating and cooling that are independently programmable for an individual substrate of a plurality of substrates; and
a controller coupled to the temperature control assembly that manages walk-away automation of the temperature control assembly and the sample processing system to automate variable temperature processing of probe micro-volumes including high temperature processing of DNA chips, protein chips, tissues, tissue micro-assays, chemical assays, biochemical assays, and biological assays, as well as Hybridization, Fluorescence In Situ Hybridization (FISH), In Situ Hybridization (ISH) assays, and other assays involving a ligand and molecular target.

FIG. 1A

FIG. 1B

FIG. 1C

FIG. 1D

FIG. 2A

FIG. 2B

FIG. 2C

FIG. 3

FIG. 4

FIG. 5A

FIG. 5B

FIG. 6A

FIG. 6B

EP 2 492 681 A1

FIG. 6C

FIG.7

FIG. 8A

FIG. 8B

FIG. 8D

FIG. 8C

FIG. 9A

FIG. 9B

FIG. 10A

FIG. 10B

FIG. 10D

FIG. 10C

FIG. 10E

FIG. 11A

EP 2 492 681 A1

610

1100

1118

1114

1104

FIG. 11B

EP 2 492 681 A1

FIG. 11C

610

1102

1112

1118

1114

1104

FIG. 11D

FIG. 11E

FIG. 11F

EP 2 492 681 A1

FIG. 11G

FIG. 12

EP 2 492 681 A1

FIG. 13

EP 2 492 681 A1

FIG. 14

Europäisches
Patentamt

European
Patent Office

Office européen
des brevets

# EUROPEAN SEARCH REPORT

**Application Number**

EP 12 15 5155

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 99/26070 A2 (MWG BIOTECH AG [DE]; HEIMBERG WOLFGANG [DE]; WEICHSELGARTNER MICHAEL []) 27 May 1999 (1999-05-27) * page 3, line 10 - page 10, line 12 * ----- | 1,2 | INV. G01N31/00 G01N33/00 G01N35/00 B01L3/00 |
| X | WO 97/39328 A1 (AUSTRALIAN BIOMEDICAL [AU]; PALANDER JARI [AU]) 23 October 1997 (1997-10-23) * page 5, line 19 - page 6, line 9; claim 1 * ----- | 1,2 | |

TECHNICAL FIELDS
SEARCHED (IPC)

B01L
G01N

The present search report has been drawn up for all claims

| Place of search | Date of completion of the search | Examiner |
|---|---|---|
| Munich | 17 July 2012 | Tragoustis, Marios |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

............................................................................

& : member of the same patent family, corresponding document

EPO FORM 1503 03.82 (P04C01)

**EP 2 492 681 A1**

This annex lists the patent family members relating to the patent documents cited in the above-mentioned European search report.
The members are as contained in the European Patent Office EDP file on
The European Patent Office is in no way liable for these particulars which are merely given for the purpose of information.

17-07-2012

| Patent document cited in search report | | Publication date | Patent family member(s) | | Publication date |
|---|---|---|---|---|---|
| WO 9926070 | A2 | 27-05-1999 | AT | 201101 T | 15-05-2001 |
| | | | DE | 29720432 U1 | 25-03-1999 |
| | | | EP | 1032839 A2 | 06-09-2000 |
| | | | JP | 4272351 B2 | 03-06-2009 |
| | | | JP | 2001523823 A | 27-11-2001 |
| | | | US | 6656724 B1 | 02-12-2003 |
| | | | WO | 9926070 A2 | 27-05-1999 |
| WO 9739328 | A1 | 23-10-1997 | EP | 0892919 A1 | 27-01-1999 |
| | | | JP | 2000508423 A | 04-07-2000 |
| | | | US | 6218191 B1 | 17-04-2001 |
| | | | WO | 9739328 A1 | 23-10-1997 |

For more details about this annex : see Official Journal of the European Patent Office, No. 12/82